# EUROPEAN PATENT APPLICATION

(11) **EP 1 134 286 A2**
(43) Date of publication of application: **19.09.2001**
(21) Application number: 01302060.7
(22) Date of filing: 06.03.2001
(51) Int. Cl.: C12N 15/57, C12N 9/64, C12N 5/10, C12N 1/21, C07K 16/40, A61K 39/395, C12Q 1/68, C12Q 1/37, A61K 38/48

(54) **Adamts polypeptides, nucleic acids encoding them, and uses thereof**

(30) Priority: 14.03.2000 US 189244 P
(71) Applicant: Pfizer Products Inc., Groton, Connecticut 06340 (US)
(72) Inventor: Buckbinder, Leonard, Groton, Connecticut 06340 (US); Mitchell, Peter Geoffrey, Groton, Connecticut 06340 (US); Wachtmann, Timothy Scott, Groton, Connecticut 06340 (US); Walsh, Roderick Thomas, Sandwich, Kent CT13 9NJ (GB)
(74) Representative: Hayles, James Richard

(57) **Abstract**

The present invention relates to a member of the family of proteins known as ADAMTS proteins, the new member being designated ADAMTS-J1. The invention also relates to polynucleotides encoding ADAMTS-J1, antibodies to ADAMTS-J1, assays for studying the function of ADAMTS-J1, assays for determining agonists or antagonists of ADAMTS-J1, and to the use of ADAMTS-J1 polypeptides or polynucleotides in diagnostic, biotherapeutic, or gene therapy methods.

## Description

### Field of Invention

The present invention relates to a member of the family of proteins known as ADAMTS.

### Background Of The Invention

ADAMTS proteins exhibit characteristics of the ADAM (A Disintegrin And Metalloprotease) family of metalloproteases, and in addition contain a thrombospondin domain (TS). The prototypic ADAMTS was identified in mouse, found to be expressed in heart and kidney and upregulated by proinflammatory stimuli (K. Kuno et al., *Molecular cloning of a gene encoding a new type of metalloproteinase-disintegrin family protein with thrombospondin motifs as a inflammation associated gene,* 272 Journal of Biological Chemistry 556 (January 1997). To date nine members are recognized by the HUGO database (http://www.gene.ucl.ac.uk/users/hester/adamts.) with sequence information available for all but two of these. Members of this family have the ability to degrade aggrecan, a high molecular weight proteoglycan which provides cartilage with important mechanical properties and which is lost during the development of arthritis.

Aggrecanase activity has been demonstrated for several ADAMTS proteins (See, e.g., M.D. Tortorella, *Purification and cloning of aggrecanase-1: a member of the ADAMTS family of proteins,* 284 Science 1664 (June 1999); I. Abbaszade, *Cloning and characterization of ADAMTS11*, *an aggrecanase from the ADAMTS family,* 274 Journal of Biological Chemistry 23443 (August 1999)). In addition to aggrecanase activity, ADAMTS-4 was shown to cleave another proteoglycan, brevican, found predominately expressed in the central nervous system (Matthews et al., *Brain-enriched hyaluronan binding (BEHAB)/Brevican cleavage in a glioma cell line is mediated by a disintegrin and metalloproteinase with thrombospondin motifs (ADAMTS) family member,* 275 Journal of Biological Chemistry 22695 (July 2000)). This activity was speculated to play a role in the invasiveness of glioma. Additional activities of ADAMTS-4 are proposed as its expression was induced in rat astrocytes treated with beta-amyloid, suggesting a role in Alzheimer's disease (Satoh et al., *ADAMTS-4 is transcriptionally induced in beta-amyloid treated rat astrocytes,* 289 Neuroscience Letters 177 (2000)). Other ADAMTS proteins are reported to exhibit antiangiogenic (See, e.g. F. Vazquez et al., *METH-1*, *A human ortholog of ADAMTS-1, and METH-2 are members of a new family of proteins with Angio-inhibitory activity,* 274 Journal of Biological Chemistry 23349 (August 1999)) and/or procollagen processing activities (A. Colige et al., *cDNA cloning and expression of bovine procollagen I N-proteinase: a* *new member of the superfamily of zinc-metalloproteinase with binding sites for cells and other matrix components,* 94 Proceedings of the National Academy of Sciences of the United States of America 2374 (March 1997)). Additional roles for ADAMTS-1 in fertility and organ development, particularly with respect to the urogenital system, were implicated by gene knock out in mice (Shindo et al" *ADAMTS-1: a metalloprotease-disintegrin essential for normal growth, fertility, and organ morphology and function,* 105 Journal of Clinical Investigation 1345 (May 2000))

ADAMTS proteins and ADAMTS protein agonists and antagonists have important therapeutic uses, including treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetic shock, infertility and other diseases characterized by metalloproteinase activity and/or characterized by mammalian adamalysin activity.

### Summary Of The Invention

This invention relates to a novel ADAMTS protein, designated ADAMTS-J1, and to related polynucleotides and polypeptides. The invention also relates to production of the protein and polypeptides and to related assays. The invention further relates to methods for identifying substrates of the protein, for identifying inhibitors or activators of the protein, and to the use of the polypeptides or polynucleotides of the invention in diagnostic, biotherapeutic, and gene therapy methods.

In particular, the invention relates to an isolated polynucleotide molecule comprising a nucleotide sequence selected from the group consisting of:
(a) a nucleotide sequence having at least 80% identity to a nucleotide sequence encoding ADAMTS-J1 polypeptide of SEQ ID NO:, 5, 6, 7 or 8, or a metalloproteinase, disintegrin domain, prodomain, or thrombospondin (TSP) domain thereof;
(b) a nucleotide sequence of at least 15 contiguous nucleotides that hybridizes under stringent conditions to the polynucleotide molecule of SEQ ID NO: 1, 2, 3 or 4; and
(c) the complement of the nucleotide sequence of (a) or (b).
Such an isolated polynucleotide molecule, can, for example, comprise DNA or RNA.

In one embodiment, the isolated polynucleotide is at least 80% identical to SEQ ID NO: 1, 2, 3, or 4, or a metalloproteinase, disintegrin domain, prodomain, or thrombospondin (TSP) domain thereof. In another embodiment the isolated polynucleotide encodes the ADAMTS-J1 polypeptide encoded by a sequence of SEQ ID NO: 1, 2, 3, or 4, or a metalloproteinase, disintegrin domain, prodomain, or thrombospondin (TSP) domain thereof. In a most. preferred embodiment, the isolated polynucleotide comprises the ADAMTS-J1 polypeptide encoding sequence of SEQ ID NO: 1, 2, 3, or 4, or a metalloproteinase, disintegrin domain, prodomain, or thrombospondin (TSP) domain encoding sequence thereof.

In a further embodiment, the invention relates to a polypeptide encoded by the isolated polynucleotide molecule of the invention. For example, the ADAMTS-J1 polypeptide of the invention can comprise an amino acid sequence which is at least 80% identical to SEQ ID NO: 5, 6, 7, or 8 or an amino acid sequence of at least about 10 contiguous amino acids of ADAMTS-J1. In a preferred embodiment, the polypeptide comprises SEQ ID NO: 5, 6, 7, or 8, or a metalloproteinase, disintegrin domain, prodomain, or thrombospondin (TSP) domain thereof.

In another aspect, the invention relates to an expression system comprising a DNA or RNA molecule, wherein the expression system is capable of producing an ADAMTS-J1 polypeptide that comprises an amino acid sequence that has at least 80% identity with a polypeptide of SEQ ID NO: 5, 6, 7, or 8, or a metalloproteinase, disintegrin domain, prodomain, or thrombospondin (TSP) domain thereof, when said expression system is present in a compatible host cell. In one embodiment of this aspect of the invention, the expression system is capable of producing an ADAMTS-J1 polypeptide encoded by a polynucleotide of the invention.

In another aspect, the invention relates to a host cell that comprises the expression system of the invention.

In another aspect, the invention relates to a process for producing an ADAMTS-J1 polypeptide that comprises culturing a host cell of the invention under conditions sufficient for production of the polypeptide, and recovering the polypeptide from cell culture.

In another aspect, the invention relates to a process for producing a cell which produces an ADAMTS-J1 polypeptide comprising transforming or transfecting a host cell with an expression system of the invention such that the host cell, under appropriate culture conditions produces the ADAMTS-J1 polypeptide.

In another aspect, the invention relates to an antibody that is immunospecific for an ADAMTS-J1 polypeptide of the invention. The invention also relates to antagonists, agonists, and substrates of the polypeptide of the invention.

In a further aspect, the invention relates to a method for treating a subject in need of altering activity or expression of ADAMTS-J1 comprising administering to the subject a therapeutically effective amount of an agonist or antagonist of ADAMTS-J1.

In another aspect, the invention relates to a method for treating a subject in need of altering activity or expression of ADAMTS-J1 comprising administering to the subject a polynucleotide of the invention in order to alter said activity or expression. The invention also relates to a method for treating a subject in need of altering activity or expression of ADAMTS-J1 comprising administering to the subject a therapeutically effective amount of a polypeptide that competes with ADAMTS-J1 for its ligand, substrate, or receptor.

The invention also relates to a process for diagnosing a disease or a susceptibility to a disease in a subject related to expression or activity of ADAMTS-J1 in a subject comprising determining presence or absence of a mutation in a nucleotide sequence encoding ADAMTS-J1 in the genome of the subject. Alternately, the invention relates to a process for diagnosing a disease or a susceptibility to a disease in a subject related to expression or activity of ADAMTS-J1 in a subject comprising analyzing for presence or amount of ADAMTS-J1 expression in a sample derived from the subject.

In another aspect, the invention relates to a method for identifying compounds which antagonize ADAMTS-J1 comprising:
(a) contacting a candidate compound with cells expressing an ADAMTS-J1 polypeptide of the invention, or with cell membranes from cells expressing the ADAMTS-J1 polypeptide, or the media conditioned by cells expressing the polypeptide, or a purified composition of said polypeptide; and
(b) determining inhibition of an ADAMTS-J1 activity.

In an alternate embodiment, the invention relates to a method for identifying compounds which agonize ADAMTS-J1 comprising:
(a) contacting a candidate compound with cells expressing an ADAMTS-J1 polypeptide of the invention, or with cell membranes from cells expressing the polypeptide, or media conditioned by cells expressing the polypeptide, or a purified composition of the polypeptide; and
(b) determining stimulation of an ADAMTS J1 activity.

In an further embodiment, the invention relates to a method for identifying compounds which bind to ADAMTS-J1 comprising:
(a) contacting a candidate compound with cells expressing an ADAMTS-J1 polypeptide of the invention, or with cell membranes from cells expressing the polypeptide, or the media conditioned by cells expressing the polypeptide, or a purified composition of the polypeptide; and
(b) determining binding of the candidate compound to the polypeptide.

The invention also relates to a method for detecting a polynucleotide encoding ADAMTS-J1 in a biological sample containing nucleic acid material comprising:
(a) hybridizing an isolated polynucleotide of the invention that is specific to ADAMTS-J1 to the nucleic acid material of the biological sample, thereby forming a hybridization complex; and
(b) detecting the hybridization complex, wherein presence of the hybridization complex correlates with the presence of the polynucleotide encoding ADAMTS-J1 in the biological sample.

In a further embodiment, the invention relates to a method for identifying a substrate for ADAMTS-J1 comprising contacting a polypeptide comprising an enzymatically active polypeptide of the invention with a candidate substrate and determining either conversion of substrate to product or binding of the polypeptide to the substrate.

The invention also relates to a method for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising administering a therapeutically effective amount of an agonist or antagonist, of ADAMTS-J1 in combination with a pharmaceutically acceptable carrier.

The invention also relates to a method for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising administering a polypeptide of the invention in combination with a pharmaceutically acceptable carrier.

The invention also relates to a method for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression; peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising administering a polynucleotide of the invention in combination with a pharmaceutically acceptable carrier.

The invention further relates to a pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a therapeutically effective amount of an agonist or antagonist, of ADAMTS-J1 in combination with a pharmaceutically acceptable carrier.

The invention also relates to a pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a polypeptide of the invention in combination with a pharmaceutically acceptable carrier.

The invention also relates to a pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a polynucleotide of the invention in combination with a pharmaceutically acceptable carrier.

### Brief Description Of The Drawings

FIGS. 1, 2, 3, and 4 show polynucleotide coding sequences of alternative splice forms of ADAMTS-J1 [SEQ ID NOS: 1, 2, 3, and 4].

FIG. 5, 6, 7, and 8 show polypeptide sequences corresponding to ADAMTS-J1 splice forms [SEQ ID NOS: 5, 6, 7, and 8].

FIG. 9 shows the partial cDNA sequence of ADAMTS-J1 identified by library screening including 3' untranslated and poly-adenylation sequences [SEQ ID NO: 9]

FIGS. 10A and B show a detailed description of the domains of ADAMTS-J1.1.

FIG. 11 shows the genomic structure of ADAMTS-J1, patterns of splicing that give rise to the four alternative forms and the corresponding domains of the ADAMTS family of proteins.

FIG. 12 shows homology of the ADAMTS-J1 polypeptide metalloprotease domain with those of other ADAMTS proteins.

FIG. 13 shows a Western Blot analysis of secreted and cell associated ADAMTS-J1-FLAG fusion proteins after transformation of HEK 293 cells with recombinant expression constructs.

### Detailed Description Of The Invention

We have found relatively high levels of polynucleotide encoding ADAMTS-J1 proteins in cDNA prepared from osteoarthritic cartilage as well as in cDNA from human brain, heart, lung and liver.

### Definitions

The following definitions are provided to facilitate understanding of terms used herein.

"Antibodies" as used herein includes polyclonal and monoclonal antibodies, chimeric, single chain, and humanized antibodies, as well as Fab fragments, including the products of a Fab or other immunoglobulin expression library.

"Polynucleotide" generally refers to any polyribonucleotide or polydeoxribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is a mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example tritylated bases and unusual bases such as inosine. A variety of modifications have been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. "Polypeptides" may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquination, and they may be cyclic, with or without branching. Cyclic, branched and branched cyclic polypeptides may result from posttranslational natural processes or may be made by synthetic methods. Modifications include acetylation, acylation, ADP-ribosylation, amidation, covalent attachment of flavin, covalent attachment of a heme moiety, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, *Proteins - structure and molecular properties,* 2nd Ed., T.E. Creighton, W.H. Freeman and Company, New York, 1993; F. Wold, *Posttranslational protein modifications: perspectives and prospects,* pgs. 1-12 in Posttranslational covalent modification of proteins, B.C. Johnson, Ed., Academic Press, New York, 1983; S. Seifter and S. Englard, *Analysis for protein modifications and nonprotein cofactors,* 182 Methods of Enzymology 626 (1990); S.I. Rattan et al., *Protein synthesis, posttranslational modifications, and aging,* 663 Ann NY Acad Sci 48 (1992).

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions and/or deletions in any combination. A substituted or inserted amino acid residue may or may not be one encoded by the genetic code. A variant of a polynucleotide or a polypeptide may be naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" per se has an art-recognized meaning and can be calculated using published techniques. See, for example, *Computational molecular biology,* A.M. Lesk, ed., Oxford University Press, New York, 1988; *Biocomputing: informatics and genome projects,* D.W. Smith, ed., Academic Press, New York, 1993; *Computer analysis of sequence data, part* 1, A.M. Griffin, and H.G. Griffin, eds., Humana Press, New Jersey, 1994; Sequence *analysis in molecular biology,* G. von Heinje, ed., Academic Press, 1987; and *Sequence analysis primer,* M. Gribskov and J. Devereux, eds., M Stockton Press, New York, 1991. While there exist a number of methods to measure identity between two polynucleotide or polypeptide sequences, the term "identity" is well known to skilled artisans. Methods to determine identity and similarity are codified in computer programs. Preferred computer program methods to determine identity and similarity between two sequences include, but are not limited to, GCS program package; J. Devereux, et al., *A comprehensive set of sequence analysis programs for the VAX,* 12(1) Nucleic Acids Research 387 (January 1984); BLASTP; BLASTN; FASTA; S.F. Altschul et al., *Basic local alignment search tool,* 215(3) Journal of Molecular Biology 403 (October 1990). Among the methods stated above to determine identity, the preferred method is BLASTP.

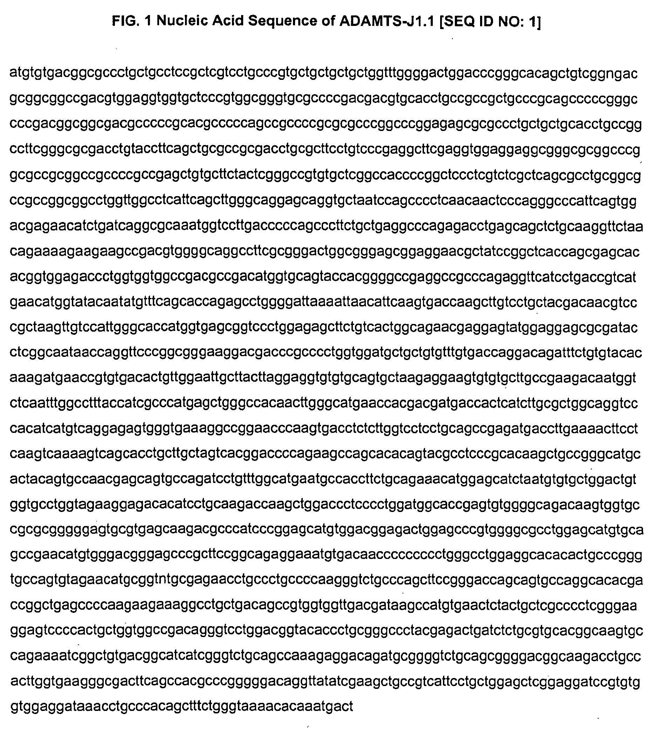

As an illustration, by a polynucleotide having a nucleotide sequence having at least, for example, 95% "identity" to a reference nucleotide sequence of FIG. 1, it is intended that the nucleotide sequence of the polynucleotide is identical to the reference sequence except that the polynucleotide sequence may include up to five point mutations per each 100 nucleotides of the reference nucleotide sequence of FIG. 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

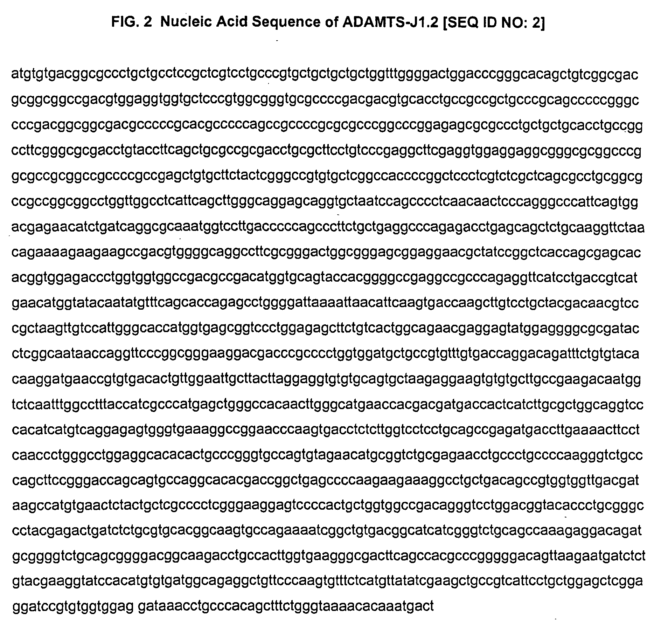

Similarly, by a polypeptide having an amino acid sequence having at least, for example, 95% "identity" to a reference amino acid sequence of FIG. 2, it is intended that the amino acid sequence of the polypeptide is identical to the reference sequence except that the polypeptide sequence may include up to five amino acid alterations per each 100 amino acids of the reference amino acid of FIG. 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### ADAMTS-J1 Polypeptides

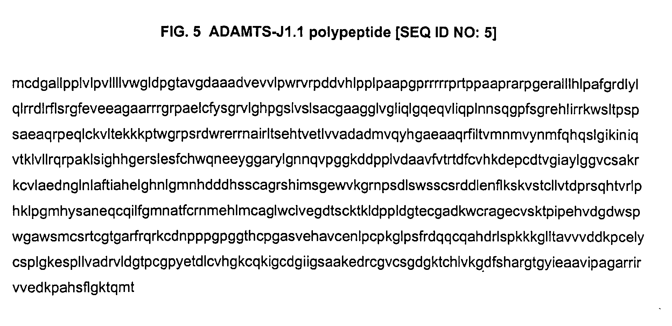

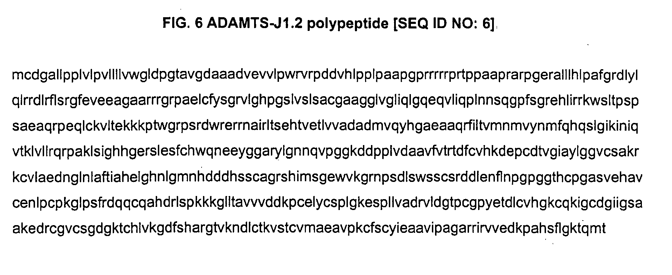

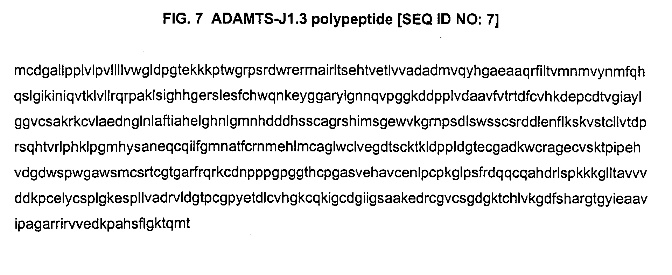

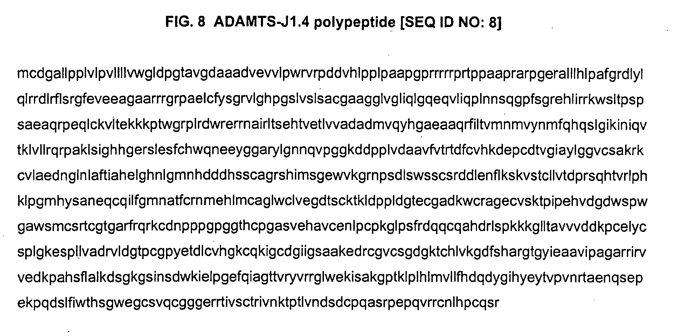

In one aspect, the present invention relates to ADAMTS-J1 polypeptides. The ADAMTS-J1 polypeptides include the polypeptides of FIG. 5-8, as well as polypeptides comprising an amino acid sequence of FIG. 5-8, and polypeptides comprising an amino acid sequence that has at least 80% identity to that of FIG. 5-8, preferably at least 90% identity, more preferably at least 95% identity to FIG. 5-8, and most preferably at least 97-99% identity to FIG. 5-8.

The ADAMTS-J1 polypeptides may be in the form of an unprocessed or partially processed precursor, or the "mature" protein, which may in turn be a part of a larger protein such as a fusion protein. The mature form should normally begin with or near amino acid 224 (beginning with NAIR...) and continue to the carboxyl terminus. It is often advantageous to include an additional amino acid sequence which contains secretory or leader sequences, pro-sequences, sequences which aid in purification or identification such as multiple histidine residues, a FLAG Tag, or an additional sequence for stability during recombinant production.

Fragments of the ADAMTS-J1 polypeptides are also included in the invention. A fragment is a polypeptide having an amino acid sequence that entirely is the same as part, but not all, of the amino acid sequence of the aforementioned ADAMTS-J1 polypeptides. As with ADAMTS-J1 polypeptides, fragments may be "free-standing," or comprised within a larger polypeptide of which they form a part or region, most preferably as a single continuous region.

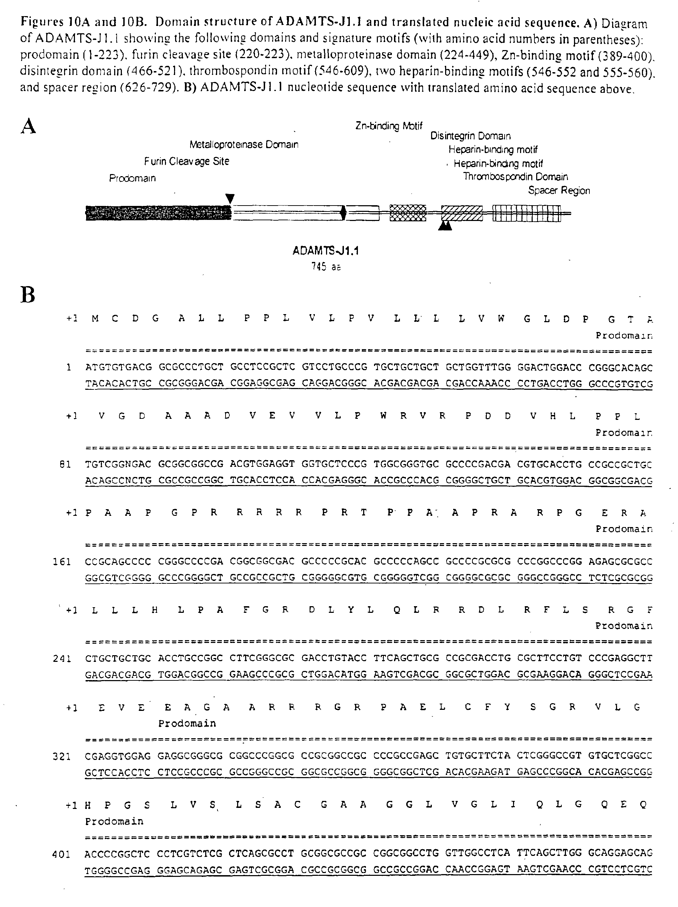

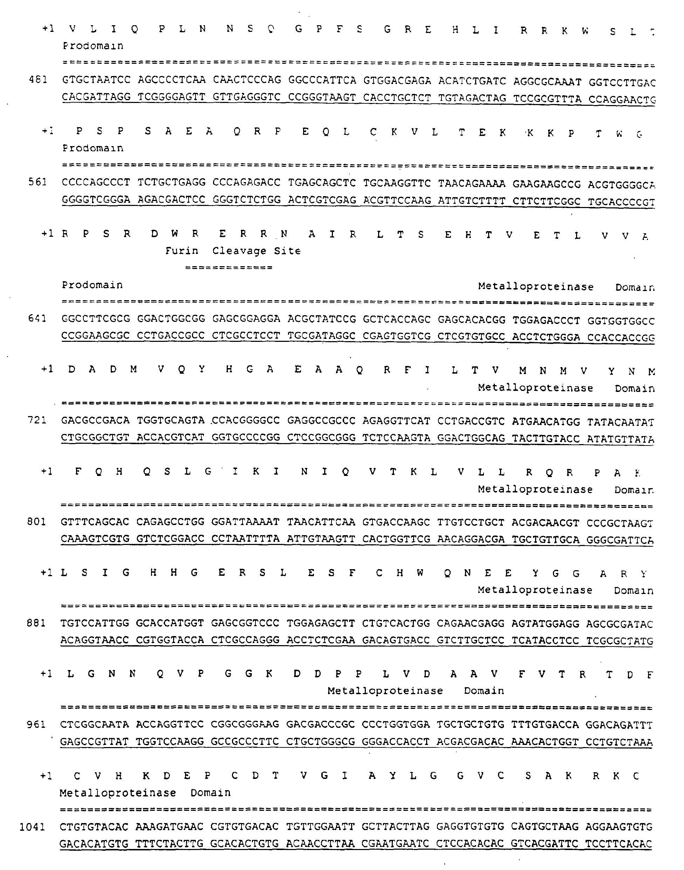

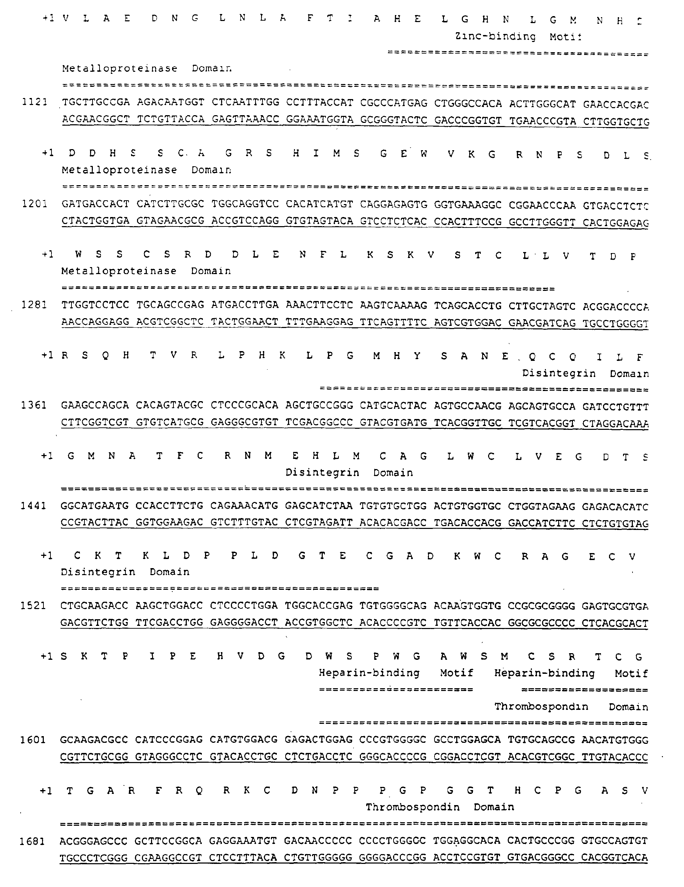

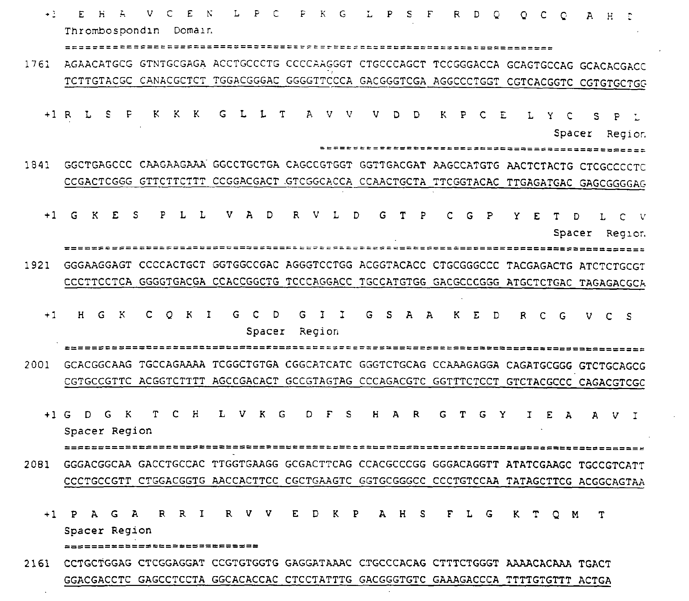

Preferred fragments include, for example, truncation polypeptides having the amino acid sequence of ADAMTS-J1 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Also preferred are biologically active fragments. Biologically active fragments are those that mediate one or more ADAMTS-J1 activities, including those with a similar activity or an improved activity, or with a decreased undesirable activity. Most preferred are fragments that comprise one or more of the domains shown in Fig. 10. In particularly preferred embodiments, the fragment comprises the metalloproteinase domain, the disintegrin domain or the thrombospondin domain. In another embodiment, the polypeptide comprises amino acids 390-413 of SEQ ID NO: 5, which encompasses an extension of the zinc binding motif.

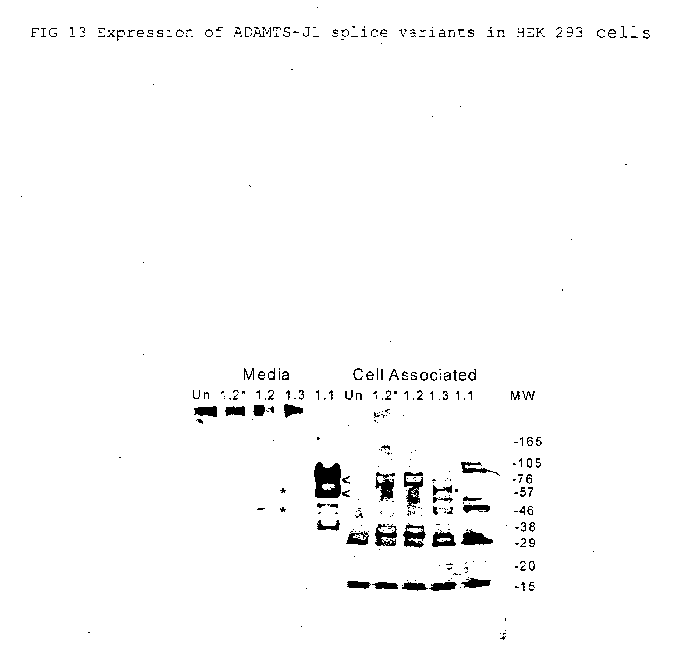

Such fragments are conventionally employed by themselves, or as part of fusion proteins. For example, expression vectors can be constructed that will express a fusion protein comprising a protein or polypeptide of the present invention. For example, as described in the Examples below, the three splice forms of ADAMTS-J1 (1.1, 1.2 and 1.3) were cloned into a mammalian expression construct (a derivative of pCDNA3.1, Invitrogen Corporation) with the addition of a FLAG epitope tag fused to the C terminus (FIG. 13). Such fusion proteins can be used, e.g., to raise antisera against the protein, to study the biochemical properties of the protein, to engineer a protein exhibiting different immunological or functional properties, to aid in the identification or purification, to improve the stability, of a recombinantly-expressed protein, or as therapeutic agents. Possible fusion protein expression vectors include but are not limited to vectors incorporating sequences that encode β-galactosidase and trpE fusions, maltose-binding protein fusions (pMal series; New England Biolabs), glutathione-S-transferase fusions (pGEX series; Pharmacia), polyhistidine fusions (pET series; Novagen Inc., Madison, WI), and thioredoxin fusions (pTrxFus; Invitrogen, Carlsbad, CA). As one example, the disintegrin domain or TSP domain, or a polypeptide comprising a variant or fragment thereof, may be administered alone, or as part of a fusion protein, to competitively inhibit in vivo or in vitro interactions with the native disintegrin domain or TSP domain. Methods are well-known in the art for constructing expression vectors encoding these and other fusion proteins.

Variants of the defined sequence and fragments also form part of the present invention. Preferred variants are those that vary from the referents by conservative amino acid substitutions, i.e., those that substitute a residue with another of like characteristics. Typical conservative substitutions are among Ala, Val, Leu and Ile; among Ser and Thr; among the acidic residues Asp and Glu; among Asn and Gln; among the basic residues Lys and Arg; and among the aromatic residues Phe and Tyr. Particularly preferred are variants in which several, 5 to 10, 1 to 5, or 1 to 2 amino acids are substituted, deleted, or added in any combination.

The ADAMTS-J1 polypeptides of the invention can be prepared in any suitable manner. The polypeptides include isolated naturally occurring polypeptides, recombinantly produced polypeptides, synthetically produced polypeptides, and polypeptides produced by a combination of these methods. These methods are well understood in the art.

Another embodiment of the present invention is an isolated ADAMTS-J1 polypeptide. An isolated polypeptide is one that has been substantially removed from its natural milieu. As isolated ADAMTS-J1 polypeptide can, for example, be obtained from its natural source, be produced using recombinant technology, or be synthesized chemically. An isolated ADAMTS-J1 polypeptide can be full-length ADAMTS-J1 polypeptide, the predicted mature form processed by furin cleavage of the prodomain (amino acid 104 with the predicted mature form beginning NAIR...), or any homologue of such a polypeptide, such as an ADAMTS-J1 polypeptide in which amino acids have been deleted, inserted, inverted, substituted and/or derivatized (e.g., by glycosylation, phosphorylation, acetylation, myristoylation, prenylation, palmitoylation, amidation and/or addition of glycosylphosphatidyl inositol). A homologue of an ADAMTS-J1 polypeptide is a polypeptide having an amino acid sequence that is sufficiently similar to a natural ADAMTS-J1 polypeptide amino acid sequence that a nucleic acid sequence encoding the homologue is capable of hybridizing under stringent conditions to a nucleic acid sequence encoding the natural ADAMTS-J1 polypeptide amino acid sequence disclosed herein. As used herein, "stringent hybridization conditions" refers to hybridization to filter-bound DNA in 0.5 M NaHPO₄, 7% SDS, 1 mM EDTA at 65°C, and washing in 0.1xSSC/0.1% SDS at 68°C (see Ausubel *et al.* (eds.), 1989, Current Protocols in Molecular Biology, Vol. I, Green Publishing Associates, Inc., and John Wiley & Sons, Inc., New York, at p. 2.10.3). A homologue of ADAMTS-J1 polypeptide also includes a polypeptide having an amino acid sequence that is sufficiently cross-reactive such that the homologue has the ability to elicit an immune response against at least one epitope of naturally-occurring ADAMTS-J1 polypeptide. Preferably the homologue retains one or more biological activities of ADAMTS-J1.

The minimal length of a protein homologue of the present invention is sufficient to be encoded by a nucleic acid molecule capable of forming a stable hybrid with the complementary sequence of a nucleic acid molecule encoding the corresponding natural protein. As such, the size of the nucleic acid molecule encoding such a protein homologue is dependent on nucleic acid composition, percent homology between the nucleic acid molecule and complementary sequence, as well as upon hybridization conditions per se (e.g., temperature, salt concentration and formamide concentration). The minimal size of such nucleic acid is typically at least about 12 to about 15 nucleotides in length if the nucleic acid molecules are GC-rich and at least about 15 to about 17 bases in length if they are AT-rich. The minimal size of a nucleic acid molecule used to encode an ADAMTS-J1 protein homologue of the present invention is from about 20 to about 25 nucleotides in length. There is no limit, other than a practical limit, on the maximal size of such a nucleic acid molecule in that the nucleic acid molecule can include a portion of a gene, an entire gene, or multiple genes, or portions thereof. In one embodiment, the minimal size of an ADAMTS-J1 protein homologue of the present invention is from 10, more preferably 12, even more preferably 25, amino acids in length. In another embodiment, a polypeptide of the invention comprises an amino acid sequence of more than about 10, or 25, preferably more than 75, more preferably more than 100, amino acids that is identical to an amino acid sequence of SEQ ID NO: 2. Preferred protein or polypeptide sizes depend on whether a full-length, multivalent protein (i.e., fusion protein having more than one domain each of which has a function), or a functional portion of such a protein is desired. Functional portions are obtainable based on the domains described herein, knowledge in the art concerning such domains and known assays for such domain, or its functional activity. Useful protein fragments or other polypeptides can also be screened for based on antigenic cross-reactivity with the ADAMTS-J1 protein of SEQ ID NO: 5-8.

ADAMTS-J1 protein homologues of the invention include allelic variations of the natural gene encoding the ADAMTS-J1 protein. A "natural" gene is that found most often in nature. ADAMTS-J1 protein homologues can be produced using techniques known in the art, including, but not limited to, direct modifications to a gene encoding a protein using, for example, classic or recombinant DNA techniques to effect random or targeted mutagenesis.

In another embodiment, an ADAMTS-J1 polypeptide of the present invention comprises a portion of the ADAMTS-J1 polypeptide disclosed herein, this portion having a molecular weight of about 25 kD (determined by Tris-glycine SDS-PAGE and resolved using methods standard in the art).

In yet another embodiment, an ADAMTS-J1 polypeptide of the present invention comprises at least a portion of an ADAMTS-J1 polypeptide encoded by a mRNA (messenger ribonucleic acid), having a length of about 681 nucleotides.

The present invention, encompasses the ADAMTS-J1 proteins that have undergone posttranslational modification. Such modification can include, for example, glycosylation (e.g., including addition of N-linked and/or O-linked oligosaccharides) or posttranslational conformational changes or posttranslational deletions.

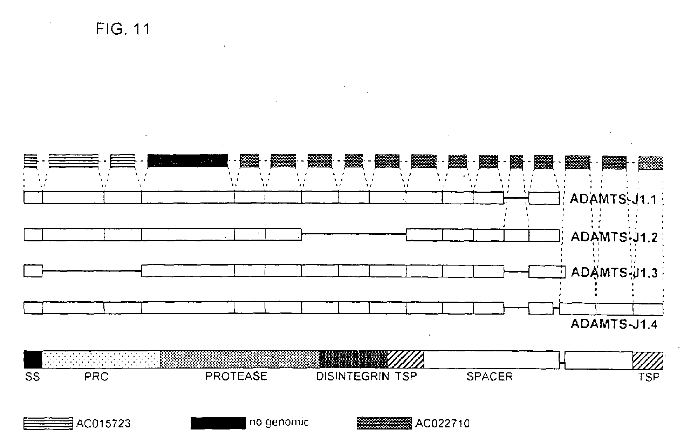

Based on the 29-36% identity in the metalloprotease domain of ADAMTS-J1 as compared to other ADAMTS family members, ADAMTS-J1 may have one or more proteolytic activities (e.g. collagenase, aggrecanase, procollagen protease) as well as anti-angiogenic activities that may or may not require the presence of the thrombospondin domains. See, FIGS. 10 and 11. These possible activities of ADAMTS-J1 can be tested using techniques known to those skilled in the art. See, e.g., P.D. Brown et al., *Independent expression and cellular processing of Mr 72,000 type IV collagenase and interstitial collagenase in human tumorigenic cell lines,* 50(19) Cancer Research 6184 (October 1990); F. Vazquez et al., *METH-1 a human ortholog of ADAMTS-1, and METH-2 are members of a new family of proteins with angio* *inhibitory activity,* 274 The Journal of Biological Chemistry 23349 (Aug. 1999); E.C. Arner et al., *Generation and characterization of* aggrecanase, 274 The Journal of Biological Chemistry 6594 (Mar. 1999); A. Colige et al., *cDNA cloning* and expression *of bovine procollagen I N-proteinase: A new member* of the *superfamily of zinc-metalloproteinases with binding sites for cell and other matrix components* 94 Proceedings of the National Academy of Sciences (USA) 2374 (March 1997).

### ADAMTS-J1 Polynucleotides

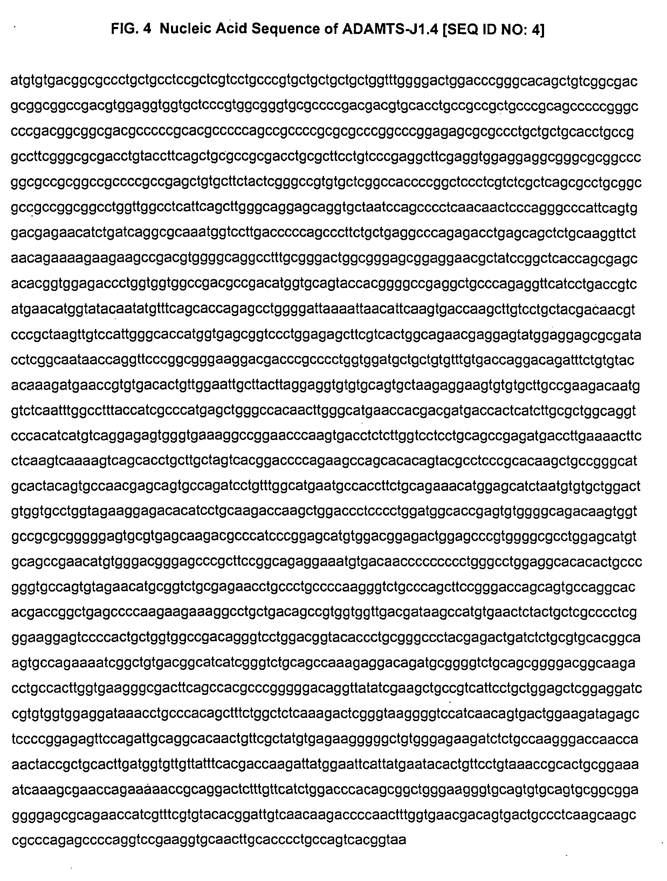

Another aspect of the invention relates to ADAMTS-J1 polynucleotides. ADAMTS-J1 polynucleotides include isolated polynucleotides which encode the ADAMTS-J1 polypeptides and fragments, and polynucleotides closely related thereto. This includes the naturally occurring splice variants identified by cloning (FIGS. 1-3) and that predicted by database searching (FIG. 4). More specifically, ADAMTS-J1 polynucleotides of the invention include a polynucleotide comprising the nucleotide sequence set forth in FIGS. 1-4 encoding a ADAMTS-J1 polypeptide of FIGS. 5-8, respectively, and a polynucleotide having the particular sequence of FIGS. 1-4. ADAMTS-J1 polynucleotides further include a polynucleotide comprising a nucleotide sequence that has at least 80% identity to a nucleotide sequence encoding the ADAMTS-J1 polypeptide of FIGS. 5-8, and a polynucleotide that is at least 80% identical to the polynucleotide sequence of FIGS. 1-4. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred.

In one embodiment, the nucleic acid molecule of the invention has a nucleotide sequence has between 1 and 50, more preferably between 1 and 25, and most preferably between 1 and 5 nucleotides inserted, deleted, or substituted with respect to the sequence of SEQ ID NOS: 1-4.

ADAMTS-J1 polynucleotides of the invention also encompass nucleotide sequences which have sufficient identity to the nucleotide sequence contained in FIGS. 1-4 to hybridize under conditions useable for amplification or for use as a probe or marker for ADAMTS-J1. Such sequences are typically 15 to 25 nucleotides in length with a target of 50% GC content and useful in PCR amplification or oligonucleotide hybridization methods well known to those skilled in the art. (See, e.g., Promega Protocols and Applications Guide, Third Edition, (1996), ISBN 1-8822474-57-1).

In one embodiment, the isolated nucleic acid molecule comprises a fragment of SEQ ID NOS: 1-4 that is specific for ADAMTS-J1, i.e., specifically acts as a probe for SEQ ID NOS: 1-4. The fragment may be at least, e.g., 15, 25, 35, 45 or 75 nucleotides in length.

Another embodiment of the present invention is an isolated nucleic acid molecule capable of hybridizing, under stringent conditions, with ADAMTS-J1 polypeptide gene (FIGS. 1-4) encoding one or more of the ADAMTS-J1 polypeptides of the present invention.

An isolated nucleic acid of the invention can include DNA, RNA or derivatives of either DNA or RNA.

An isolated nucleic acid molecule of the present invention can be obtained from its natural source either as an entire (i.e., complete) gene or a portion thereof capable of forming a stable hybrid with that gene. As used herein, the phrase "at least a portion of" an entity refers to an amount of the entity that is at least sufficient to have functional aspects of that entity. For example, at least a portion of a nucleic acid sequence, as used herein, is an amount of a nucleic acid sequence capable of forming a stable hybrid with a particular desired gene (e.g., ADAMTS genes) under stringent hybridization conditions. An isolated nucleic acid molecule of the present invention can also be produced using recombinant technology (e.g., polymerase chain reaction (PCR) amplification, cloning) or chemical synthesis. Isolated ADAMTS-J1 protein nucleic acid molecules include natural nucleic acid molecules and homologues thereof, including, but not limited to natural allelic variants and modified nucleic acid molecules in which nucleotides have been inserted, deleted, substituted, and/or inverted in a manner that does not substantially interfere with the nucleic acid molecule's ability to encode an ADAMTS-J1 protein of the present invention or to form stable hybrids under stringent conditions with natural nucleic acid molecule isolates encoding an ADAMTS-J1 protein.

The invention also provides polynucleotides that are complementary to ADAMTS-J1 polynucleotides described above.

### Expression of ADAMTS-J1

In one embodiment, an isolated ADAMTS-J1 protein of the present invention is produced by culturing a recombinant cell capable of expressing the protein under conditions effective to produce the protein, and recovering the protein. Preferred cells include bacterial (e.g., *E. coli*), yeast (e.g., *Pichia),* insect (e.g., SF9) or mammalian cells (e.g., CHO, Cos 7, and HEK 293). The recombinant cell is capable of expressing the ADAMTS-J1 protein and is produced by transforming a host cell with one or more nucleic acid molecules of the present invention. Such recombinant cells are part of the present invention. Suitable transformation techniques include, but are not limited to, transfection, electroporation, microinjection, lipofection, adsorption and protoplast fusion. Recombinant cells of the invention may remain unicellular or may grow into a tissue organ or a multicellular organism. Nucleic acid molecules of the present invention used to transformed cells according to conventional techniques can remain extrachromosomal or can integrate into one or more sites within a chromosome of the transformed (i.e., recombinant) cell in such a manner that their ability to be expressed is retained.

Suitable host cells for transforming a cell include any cell capable of producing ADAMTS-J1 proteins of the present invention after being transformed with at least one nucleic acid molecule of the present invention. Host cells can be either untransformed cells or cells that are already transformed with at least one nucleic acid molecule the present invention. Suitable host cells include bacterial, fungal (including yeast), insect, animal and plant cells.

The present invention also encompasses a recombinant vector which comprises a polynucleotide of the present invention inserted into a vector capable of delivering the polynucleotide into a host cell. Such a vector normally contains heterologous nucleic acid sequences, for example nucleic acid sequences that are not naturally found adjacent to ADAMTS-J1 protein nucleic acid molecules of the present invention. The vector can be either DNA or RNA, and either prokaryotic or eukaryotic, and is typically a virus or a plasmid. Recombinant vectors can be used in cloning, sequencing, and/or otherwise manipulating or expressing ADAMTS-J1 polynucleotides of the present invention.

In one embodiment of the invention, a recombinant cell is produced by transforming a host cell with one or more recombinant molecules, each comprising one or more polynucleotide molecules of the present invention operatively linked to an expression vector containing one or more transcription control sequences. The phrase "operatively linked" refers to a nucleic acid molecule inserted into an expression vector in a manner such that the molecule is able to be expressed when transformed into a host cell. As used herein, the phrase "expression vector" refers to a DNA or RNA vector that is capable of transforming a host cell and of effecting expression of a specified nucleic acid molecule.

Preferably, the expression vector is also capable of replicating within the host cell. Expression vectors can be either prokaryotic or eukaryotic, and are typically viruses or plasmids. Expression vectors of the present invention include vectors that effect direct gene expression in bacterial, fungal, insect, animal, and/or plant cells. Nucleic acid molecules of the present invention can be operatively linked to expression vectors containing regulatory sequences such as promoters, operators, repressors, enhancers, termination sequences, origins of replication, and other regulatory sequences that are compatible with the recombinant cell and that control the expression of nucleic acid molecules. Transcription control sequences that can be used in the present invention include those capable of controlling the initiation, elongation, and termination of transcription. Particularly important transcription control sequences are those which control transcriptional initiation, such as promoter, enhancer, operator and repressor sequences. Suitable transcription control sequences include those that function in one of the recombinant cells of the present invention. A variety of such transcription control sequences are known to those skilled in the art. Preferred transcription control sequences include those which function in bacterial yeast and mammalian cells, such as, but not limited to, tac, lac, trp, trc, oxy-pro, omp/lpp, rmB, bacteriophage lambda (λ) (such as λ_{P} and λP_{R} and fusions that include such promoters), bacteriophage T7, T7lac, bacteriophage T3, bacteriophage SP6, bacteriophage SPO1, metallothionein, alpha mating factor, baculovirus, vaccinia virus, herpesvirus, poxvirus, adenovirus, simian virus 40, retrovirus action, retroviral long terminal repeat, Rous sarcoma virus, heat shock, phosphate and nitrate transcription control sequences, as well as other sequences capable of controlling gene expression in prokaryotic or eukaryotic cells. Additional suitable transcription control sequences include tissue-specific promoters and enhancers as well as lymphokine-inducible promoters (e.g., promoters inducible by interferons or interleukins). Transcription control sequences useful in practicing the present invention include naturally occurring sequences associated with DNA encoding an ADAMTS-J1 protein.

Preferred nucleic acid molecules for insertion into an expression vector include nucleic acid molecules encoding at least a portion of an ADAMTS-J1 protein, or a homologue thereof. Expression vectors of the present invention may also contain fusion sequences, e.g., as discussed above, which allow expression of nucleic acid molecules of the present invention as fusion proteins. Inclusion of a fusion sequence in an ADAMTS-J1 nucleic acid molecule of the present invention can enhance stability during production, storage, or use of the protein encoded by the nucleic acid molecule. Furthermore, a fusion segment can simplify detection and purification of an ADAMTS-J1 protein, enabling purification by affinity chromatography. Fusion segments can be of any size that affords the desired function (e.g., increased stability and/or easier purification). It is within the scope of the present invention to use one or more fusion segments. Fusion segments can be joined to amino and/or carboxyl termini of the ADAMTS-J1 protein or polypeptide of the present invention. Linkages between fusion segments and ADAMTS-J1 proteins can be constructed to be susceptible to cleavage to enable straightforward recovery of the ADAMTS-J1 proteins. Fusion proteins are preferably produced by culturing a recombinant cell transformed with nucleic acid sequences that encode the fusion segment attached to either the carboxyl and/or amino terminal end of a ADAMTS-J1 polypeptide of the invention.

The present invention includes recombinant cells resulting from transformation with a nucleic acid molecule of the present invention. Preferred recombinant cells are transformed with a nucleic acid molecule that encodes at least a portion of an ADAMTS-J1 protein, or a homologue thereof. Amplifying the copy number of nucleic acid sequences of the invention can be accomplished by increasing the copy number of the nucleic acid sequence in the cell's genome or by introducing additional copies of the nucleic acid sequence by transformation. Copy number amplification is conducted in a manner such that greater amounts of enzyme are produced, leading to enhanced conversion of substrate to product. Transformation can be accomplished using any process by which nucleic acids are transformed into cells to enhance enzyme synthesis. Prior to transformation, the nucleic acid sequence can, if desired, be manipulated to encode an enzyme having a higher specific activity.

In accordance with the present invention, recombinant cells are used to produce an ADAMTS-J1 protein of the present invention by culturing such cells under conditions effective to produce such a protein, and the protein recovered. Effective conditions include, but are not limited to, appropriate media, bioreactor, temperature, pH and oxygen conditions that permit protein production. Suitable media are typically aqueous and comprise assimilable carbohydrate, nitrogen and phosphate sources, as well as appropriate salts, minerals, metals and other nutrients, such as vitamins. The medium may comprise complex nutrients, or may be minimal.

Cells of the present invention can be cultured in conventional fermentation bioreactors, which include, but are not limited to, batch, fed-batch, cell recycle, and continuous fermentors. Culturing can also be conducted in shake flasks, test tubes, microtiter dishes, and petri plates. Culturing is carried out at a temperature, pH and oxygen content appropriate for the recombinant cell. Such culturing is within the expertise of one of ordinary skill in this art.

Depending on the vector and host system used for production, resultant ADAMTS-J1 proteins may either remain within the recombinant cell or be secreted into the fermentation medium. "Recovering the protein" according to the invention may involve simply collecting the fermentation medium or cells containing the protein and need not include additional steps of separation or purification. ADAMTS-J1 proteins of the present invention can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, chromatofocusing and differential solubilization.

In addition, an ADAMTS-J1 protein of the present invention can be produced by isolating the ADAMTS-J1 protein from cells expressing the ADAMTS-J1 protein recovered from transgenic animal, or from fluid, such as milk, recovered from such an animal. An isolated protein or polypeptide of the present invention can be used to formulate a therapeutic composition as discussed further below.

### Antibodies to ADAMTS-J1

The present invention also includes antibodies capable of selectively binding to an ADAMTS-J1 protein or polypeptide of the present invention. Polyclonal populations of anti-ADAMTS-J1 antibodies can be contained in an ADAMTS-J1 antiserum. Binding can be measured using a variety of methods known to those skilled in the art including immunoblot assays, immunoprecipitation assays, enzyme immunoassays (e.g., ELISA), radioimmunoassay, immonofluorescent antibody assays and immunoelectron microscopy; see, for example, Sambrook et al., *Molecular cloning: a laboratory manual,* Cold Spring Harbor Labs Press, 1989.

Antibodies of the present invention can be either monoclonal or polyclonal antibodies and can be prepared using techniques standard in the art. Antibodies of the present invention include functional equivalents such as antibody fragments and genetically-engineered antibodies, including single chain antibodies that are capable of selectively binding to at least one of the epitopes of the protein used to obtain antibodies. Preferably, antibodies are raised in response to proteins that are encoded, at least in part, by an ADAMTS-J1 nucleic acid molecule.

### Identification of ADAMTS-J1 substrates

The present invention also encompasses methods for identifying ADAMTS-J1 substrates. Such methods include those wherein a candidate substrate is contacted with a polypeptide comprising an enzymatically active ADAMTS-J1 polypeptide of the invention, and conversion of substrate to product is determined, or binding of polypeptide to the candidate substrate determined. The invention also encompasses rational drug design conducted using computer software that calculates interactions between candidate compounds and polypeptides or polynucleotides of the invention.

Substrates may be identified by a candidate protein or synthetic substrate approach. For example, candidate proteins can be cast within an agarose gel matrix and the ability of the ADAMTS-J1 protein to digest the protein determined using protein zymography. See, P.D. Brown et al., *Independent expression and cellular processing of Mr 72,000 type IV collagenase and interstitial collagenase in human tumorigenic cell lines,* 50(19) Cancer Research 6184 (October 1990). Alternatively, a phage display or fluorometric peptide library can be screened to identify substrates of the protein. See, D.R. O'Boyle et al., *Identification of a novel peptide substrate of HSV-1 protease using substrate phage display,* 236(2) Virology 338 (September 1997).

### Agonists/antagonists of ADAMTS-J1

The present invention also includes assays for determining agonists and/or antagonists of ADAMTS-J1. Assays for determining aggrecanase, collagenase, procollagen protease and/or angiogenic activities may be used to identify agonist or antagonist compounds, preferably small molecular weight compounds of less than 700 daltons. The compounds may contain a hydroxamic acid moiety or an optionally substituted heterocyclic nucleus, or an aryl or heteroaryl sulfonamide moiety, which compounds inhibit or stimulate the activity of endogenous or recombinant ADAMTS-J1. E.C. Arner et al., *Generation and characterization of aggrecanase. A soluble, cartilage-derived aggrecan-degrading activity,* 274 Journal of Biological Chemistry 6594 (March 1999); M.D. Tortorella et al., *supra;* A. Colige et al., *supra;* K. Kuno et al., *supra.* ELISA or fluorescent substrate assays can be performed to determine agonists or antagonists, or to determine specific proteolytic activity, of an ADAMTS-J1 protein.

### Diagnostic Assays

The present invention also includes processes for diagnosing diseases or susceptibility to diseases related to expression and/or activity of ADAMTS-J1. Such diseases may be identified by determining the presence or absence of a mutation in the nucleotide sequence encoding said ADAMTS-J1 polypeptide in the genome of a patient. Alternately, the presence or amount of ADAMTS-J1 polypeptide in a sample derived from a patient may be determined as an indicator of disease or susceptibility to disease. Such diagnosis may be performed for diseases including the following: arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetic shock, infertility and other diseases characterized by metalloproteinase activity and/or characterized by mammalian adamalysin activity.

### Therapeutic compositions and uses of ADAMTS-J1

In one embodiment of the present invention, an antibody, agonist, antagonist, substrate and/or variant of ADAMTS-J1, or a polypeptide or polynucleotide of the invention, is employed in a therapeutic composition for treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetic shock, infertility or other diseases characterized by metalloproteinase activity and/or mammalian adamalysin activity.

In one embodiment, polynucleotides of the invention can, for example, be employed to transform cells in gene therapy application, e.g., as part of in vivo or ex vivo gene therapy. Polynucleotides can also be employed in antisense therapy, and in the construction of ribozymes. Use of polynucleotides in these methods is known to those skilled in this art.

For administration to mammals, including humans, a variety of conventional routes may be used including oral, parenteral (e.g., intravenous, intramuscular or subcutaneous), buccal, anal and topical.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelation and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof. In the case of animals, they are advantageously contained in an animal feed or drinking water in a concentration of 5-5000 ppm, preferably 25 to 500 ppm.

For parenteral administration (intramuscular, intraperitoneal, subcutaneous and intravenous use) a sterile injectable solution of the active ingredient is usually prepared. Solutions of the therapeutic compound in either sesame or peanut oil or in aqueous propylene glycol may be employed. The aqueous solutions should be suitably adjusted and buffered, preferably at a pH of greater than 8, if necessary and the liquid diluent first rendered isotonic. These aqueous solutions are suitable intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art. In the case of animals, compounds can be administered intramuscularly or subcutaneously at dosage levels of about 0.1 to 50 mg/kg/day, advantageously 0.2 to 10 mg/kg/day given in a single dose or up to 3 divided doses.

Active compounds may also be formulated in rectal compositions such as suppositories or retention enemas, e.g., containing conventional suppository bases such as cocoa butter or other glycerides.

For intranasal administration or administration by inhalation, active compounds are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A therapeutic composition of the present invention can be administered to any subject having a medical disorder as herein described. Acceptable protocols by which to administer therapeutic compounds of the present invention in an effective manner vary according to individual dose size, number of doses, frequency of dose administration and mode of administration. Determination of appropriate protocols is accomplished by those skilled in the art without undue experimentation. An effective dose refers to a dose capable of treating a subject for a medical disorder as described herein. Effective doses vary depending upon, for example, the therapeutic composition used, the medical disorder being treated and the size and type of recipient animal.

The dosage and length of treatment depends on the disease state being treated. The duration of treatment may be a day, a week or longer and may last over the lifetime of the patient.

### Identification of ADAMTS-J1

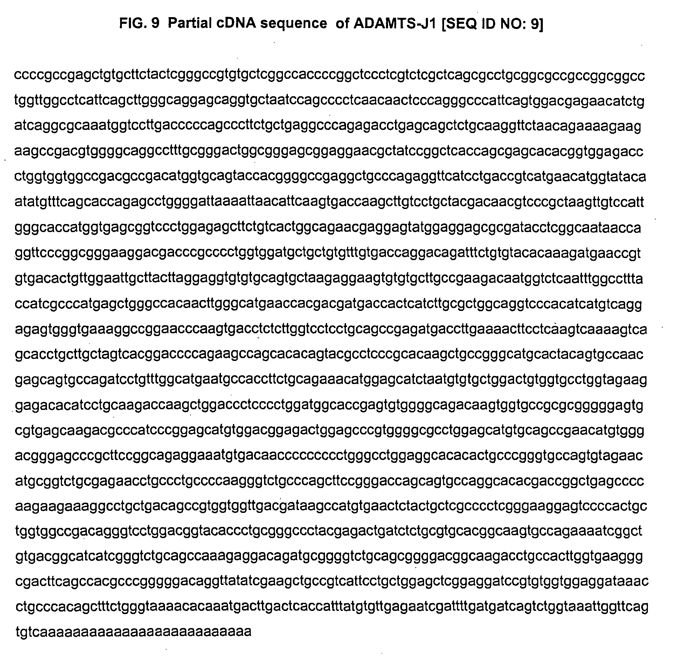

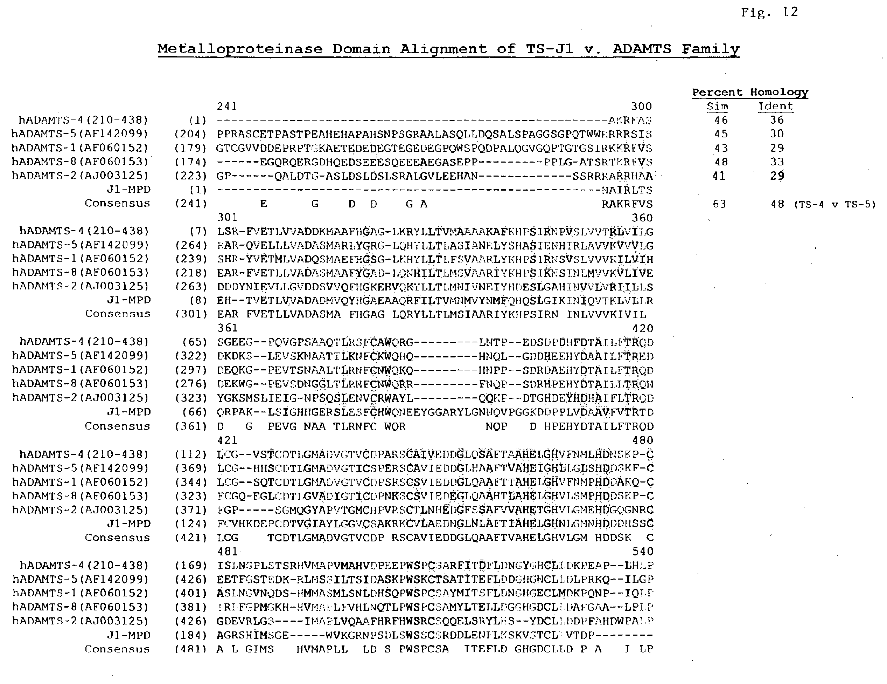

For identification of novel ADAMTS gene family members, a non-redundant set of publicly available protein sequences was assembled (accession numbers: D67076, AJ003125, AB002364, AB014588). A series of low stringency BLAST searches were then performed against the LifeSeq Gold™ database (Incyte) using each of the above protein sequences as queries. A 278bp EST was identified containing a Zn-binding motif and upstream sequence. This sequence was used to design PCR primers, and a panel of libraries was screened to determine a source for further cloning. An abundant product was obtained from an osteoarthritic cartilage cDNA library, which was then screened by RecA-mediated homology capture. Colonies hybridizing to the capture sequence were isolated, and miniprep DNA was used for PCR with outer primers. Two positive clones were identified, one of which was sequenced in its entirety (FIG. 9). BLASTP 2.0.9 analysis showed high homology to a number of ADAMTS family members, as indicated in the alignment in Figure 12.

In order to identify the 5' end of the gene, the HTG (high throughput genomic) section of Genbank was searched with the sequence obtained. A match to the start of this sequence was found in a 32kb contig in the entry AC015723. To identify the 5' prime exons two gene prediction tools (Genscan and Genie) were used on the genomic contig. Exons which showed some homology to known ADAMTSs and were of appropriate size and position were predicted to be the start of the coding region.

The 3' end of the sequence was found to match to the HTG entry AC022710. By comparing the genomic sequence to known ADAMTSs three exons with homology to the 3' end of ADAMTSs were predicted beyond the final exon of the ADAMTS-J1 sequence. This potential extended splice variant was confirmed by identification of a cDNA, the Incyte EST 4739224F7, which covers the 3' end of the ADAMTS-J1 sequence except for an alternate splice site at the final exon and continues into the 3 extra predicted exons.

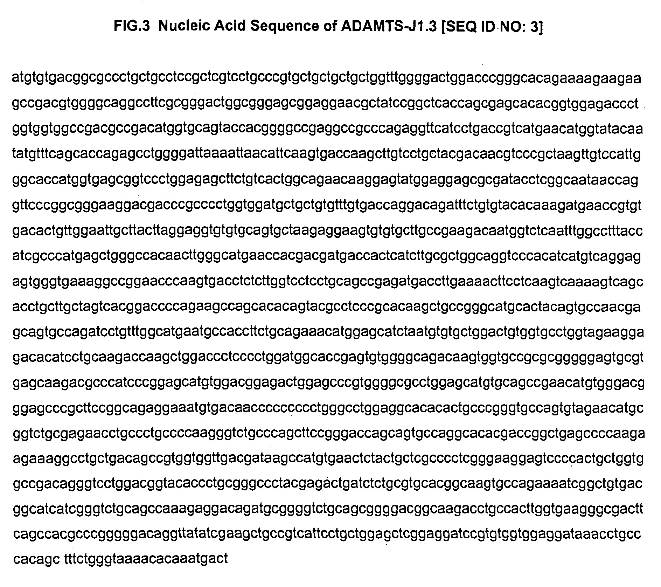

In order to generate the 5' cDNA predicted by analysis of the genomic sequence, but missing from the library clone [FIG. 9] an RT-PCR strategy was utilized. RNA from human OA chondrocytes as well as from adult and fetal brain (CloneTech Inc., Palo Alto, CA) were used as templates. First strand cDNA was primed with anti-sense primers to the 3' end [primer J05: tca ctt gtc atc gtc gtc ctt gta gtc ACC CAG AAA GCT GTG GGC A; (SEQ ID NO: 10) lower case nucleotides correspond to the addition of a FLAG tag] or from the middle [primer J01: GGA CGT TGT CGT AGC AGG AC] (SEQ ID NO: 11) of the gene. The 5' PCR primer [J02: gga att CGG GTA CCA TGT GTG ACG GC (SEQ ID NO: 12); lower case nucleotides add an EcoRI restriction site]. Products from RT-PCR reactions were cloned and nucleotide sequence determined. The missing 5' end from the genomic clone was found to be expressed in fetal brain [see FIG. 1, SEQ ID NO: 1, showing the nucleic acid sequence of ADAMTS-J1.1], while an alternative 5' splice was identified in both OA chondrocytes and fetal brain RNA [see FIG. 3, SEQ ID NO: 3, showing the nucleic acid sequence of ADAMTS-J1.2]. The alternative 3' splice was found to be expressed in fetal brain [see FIG. 2, SEQ ID NO: 2, showing the nucleic acid sequence of ADAMTS-J1.3]. FIG. 4, SEQ ID NO: 4 shows the nucleic acid sequence of an alternative splice variant, ADAMTS-J1.4,that was identified. Corresponding amino acid sequences for the splice variants are shown in FIGS. 5-8. A qualitative PCR analysis was consistent with the expression of ADAMTS-J1 forms in human OA chondrocytes, adult and fetal brain, heart, liver and kidney.

FIGS. 10A and B show a detailed description of the domains of ADAMTS-J1.1. The sequences of the domains of ADAMTS-J1.2, 1.2, 1.3,and 1.4 can be easily found by identifying the corresponding regions of 1.1.

FIG. 11 shows the genomic structure of ADAMTS-J1, patterns of splicing that give rise to the four alternative forms and the corresponding domains of the ADAMTS family of proteins. In this figure, "PRO" indicates prodomain, "TSP" indicates the thrombospondin domain, "SS" indicates signal sequence, and "no genomic" indicates that no genomic sequence was found for the sequence spanning the indicated region.

### Expression of ADAMTS-J1 Forms

Mammalian expression constructs corresponding to the three splice forms of ADAMTS-J1 and containing a FLAG-epitope tag were prepared by cloning the PCR products into a pCDNA3.1-based vector (Invitrogen, Carlsbad, CA). Sequence verified plasmids were transfected into HEK 293 cells (ATCC) using a cationic lipid, LF2000 (Life Technologies Inc., Gaithersburg, MD). Eight hours post transfection cells were changed into IS293 media (Irvine Scientific, Irvine, CA). After 48 hours media was collected and cellular proteins harvested in 2X Tris-Glycine Sample buffer (Novex). Media was concentrated by acid precipitation (10% final TCA).

Expression of the secreted (1x10⁶ cell equivalents) and cell-associated (1x10⁵ cells) proteins were analyzed by Western blot. In particular, samples and prestained/biotinylated molecular weight markers were resolved by SDS-PAGE (4-20% tris-glycine, Novex), and transferred to nitrocellulose membrane. FLAG-reactive proteins were identified by incubating the Western blots with an HRP-conjugated anti-FLAG antibody (M2, Sigma Inc., St. Louis, MO) and visualized by chemiluminesence (Super Signal, Piercer Inc.) and imaging on a Lumilmager (Roche Diagnostics, Indianapolis, IN).

Consistent with the predicted secreted (80 kD) and furin processed (58 kD) forms of 1.1, FLAG reactive products were detected in the media of transfected cells as indicated in FIG. 13 by the (<). Likewise, consistent with the predicted secreted (67 kD) and furin processed (45 kD) forms of 1.2, FLAG reactive products were detected in the media (*). No FLAG reactivity was detected in mock transfected cells (Un), those transfected with a construct expressing 1.3 or a form of 1.2* with multiple point mutations. FLAG reactive products specific to cells transfected with each splice form were identified in the analysis of cellular proteins, including the splice form 1.3 (■).

These results demonstrate that splice forms 1.1 and 1.2 give rise to secreted products of the expected molecular weight for secreted and furin processed polypeptides (FIG. 13). In addition, and consistent with observations in baculovirus expression systems, a construct expressing a form lacking most of the prodomain (splice form 1.3 and data not shown) fails to demonstrate the presence of a secreted product, but does show production of a cell associated product of approximately the correct size (FIG. 13). These results indicate that a prodomain is necessary for proper expression of secreted forms, and suggest function for the cell associated form, i.e., 1.3.

The foregoing description of the invention has been presented for purposes of illustration and description. Further, the description is not intended to limit the invention to the form disclosed herein. Consequently, variations and modifications commensurate with the above teachings, and the skill or knowledge in the relevant art are within the scope of the present invention. It is intended that the appended claims be construed to include alternate embodiments to the extent permitted by the prior art.

### Sequence ID Listing

SEQ ID NOS: 1-4 are the nucleotide sequences of ADAMTS-J1 splice forms.

SEQ ID NOS: 5-8 are the deduced amino acid sequences of ADAMTS-J1 forms.

SEQ ID NO: 9 is the nucleotide sequence of the partial ADAMTS-J1 cDNA clone identified by library screening.

SEQ ID NOS. 10-12 are nucleotide sequences of PCR primers described herein.

## Claims

1. An isolated polynucleotide molecule comprising a nucleotide sequence selected from the group consisting of:
(a) a nucleotide sequence having at least 80% identity to a nucleotide sequence encoding an ADAMTS-J1 polypeptide of SEQ ID NO: 5, 6,7, or 8, or a metalloproteinase, disintegrin domain, prodomain, or thrombospondin (TSP) domain thereof;
(b) a nucleotide sequence of at least 15 contiguous nucleotides that hybridizes under stringent conditions to a polynucleotide molecule of SEQ ID NO: 1, 2, 3, or 4; and
(c) the complement of the nucleotide sequence of (a) or (b).

2. An isolated polynucleotide molecule of claim 1, wherein said polynucleotide sequence comprises the ADAMTS-J1 polypeptide encoding sequence of SEQ ID NO: 1, 2, 3, or 4, or a metalloproteinase, disintegrin domain, prodomain, or thrombospondin (TSP) domain encoding sequence thereof.

3. A polypeptide encoded by the isolated polynucleotide molecule of claim 1.

4. The polypeptide of claim 3 which comprises an amino acid sequence that is SEQ ID NO: 5, 6, 7, or 8, or a metalloproteinase, disintegrin domain, prodomain, or thrombospondin (TSP) domain thereof.

5. An expression system comprising a DNA or RNA molecule, wherein said expression system is capable of producing an ADAMTS-J1 polypeptide of claim 3 when said expression system is present in a compatible host cell.

6. A host cell comprising the expression system of claim 5.

7. A process for producing an ADAMTS-J1 polypeptide comprising culturing a host cell of claim 6 under conditions sufficient for production of said polypeptide, and recovering the polypeptide from cell culture.

8. An agent selected from the group consisting of an antibody immunospecific for an ADAMTS-J1 polypeptide, an agonist for an ADAMTS-J1 polypeptide, an antagonist for an ADAMTS-J1 polypeptide, and a substrate for an ADAMTS-J1 polypeptide, wherein said polypeptide is the polypeptide of claim 4.

9. Use of an agent according to claim 8 in the manufacture of a medicament for treating a subject in need of altering activity or expression of ADAMTS-J1.

10. A process for diagnosing a disease or a susceptibility to a disease in a subject related to expression or activity of ADAMTS-J1 in a subject comprising determining presence or absence of a mutation in a nucleotide sequence encoding a polypeptide of claim 3 in the genome of said subject, or analyzing for presence or amount of ADAMTS-J1 expression in a sample derived from said subject.

11. A method for identifying compounds which antagonize, agonize, or bind to ADAMTS-J1 comprising:
(a) contacting a candidate compound with cells expressing an ADAMTS-J1 polypeptide of claim 3, or with cell membranes from cells expressing said ADAMTS-J1 polypeptide, or the media conditioned by cells expressing said polypeptide, or a purified composition of said polypeptide; and
(b) determining inhibition or stimulation of an ADAMTS-J1 activity, or binding or said candidate compound to said polypeptide.

12. A method for detecting a polynucleotide encoding ADAMTS-J1 in a biological sample containing nucleic acid material comprising:
(a) hybridizing an isolated polynucleotide of claim 1 that is specific to ADAMTS-J1 to the nucleic acid material of the biological sample, thereby forming a hybridization complex; and
(b) detecting the hybridization complex, wherein presence of the hybridization complex correlates with the presence of the polynucleotide encoding ADAMTS-J1 in the biological sample.

13. A method for identifying a substrate for ADAMTS-J1 comprising contacting a polypeptide comprising an enzymatically active polypeptide of claim 3 with a candidate substrate and determining either conversion of substrate to product or binding of the polypeptide to the substrate.

14. Use of an agent selected from the group consisting of an agonist or antagonist of ADAMTS-J1, a polypeptide of claim 3, and a polynucleotide of claim 1, in the manufacture of medicament for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock.

15. A pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock, comprising a therapeutically effective amount of an agent selected from the group consisting of an agonist or antagonist of ADAMTS-J1, a polypeptide of claim 3, and a polynucleotide of claim 1, in combination with a pharmaceutically acceptable carrier.


SEQUENCE LISTING

<110> Pfizer Products Inc.

<120> ADAMTS POLYPEPTIDES, NUCLEIC ACIDS ENCODING THEM, AND
      USES THEREOF

<130> PC10736A

<140>
<141>

<160> 12

<170> PatentIn Ver. 2.1

<210> 1
<211> 2235
<212> DNA
<213> Human

<400> 1
atgtgtgacg gcgccctgct gcctccgctc gtcctgcccg tgctgctgct gctggtttgg 60
ggactggacc cgggcacagc tgtcggngac gcggcggccg acgtggaggt ggtgctcccg 120
tggcgggtgc gccccgacga cgtgcacctg ccgccgctgc ccgcagcccc cgggccccga 180
cggcggcgac gcccccgcac gcccccagcc gccccgcgcg cccggcccgg agagcgcgcc 240
ctgctgctgc acctgccggc cttcgggcgc gacctgtacc ttcagctgcg ccgcgacctg 300
cgcttcctgt cccgaggctt cgaggtggag gaggcgggcg cggcccggcg ccgcggccgc 360
cccgccgagc tgtgcttcta ctcgggccgt gtgctcggcc accccggctc cctcgtctcg 420
ctcagcgcct gcggcgccgc cggcggcctg gttggcctca ttcagcttgg gcaggagcag 480
gtgctaatcc agcccctcaa caactcccag ggcccattca gtggacgaga acatctgatc 540
aggcgcaaat ggtccttgac ccccagccct tctgctgagg cccagagacc tgagcagctc 600
tgcaaggttc taacagaaaa gaagaagccg acgtggggca ggccttcgcg ggactggcgg 660
gagcggagga acgctatccg gctcaccagc gagcacacgg tggagaccct ggtggtggcc 720
gacgccgaca tggtgcagta ccacggggcc gaggccgccc agaggttcat cctgaccgtc 780
atgaacatgg tatacaatat gtttcagcac cagagcctgg ggattaaaat taacattcaa 840
gtgaccaagc ttgtcctgct acgacaacgt cccgctaagt tgtccattgg gcaccatggt 900
gagcggtccc tggagagctt ctgtcactgg cagaacgagg agtatggagg agcgcgatac 960
ctcggcaata accaggttcc cggcgggaag gacgacccgc ccctggtgga tgctgctgtg 1020
tttgtgacca ggacagattt ctgtgtacac aaagatgaac cgtgtgacac tgttggaatt 1080
gcttacttag gaggtgtgtg cagtgctaag aggaagtgtg tgcttgccga agacaatggt 1140
ctcaatttgg cctttaccat cgcccatgag ctgggccaca acttgggcat gaaccacgac 1200
gatgaccact catcttgcgc tggcaggtcc cacatcatgt caggagagtg ggtgaaaggc 1260
cggaacccaa gtgacctctc ttggtcctcc tgcagccgag atgaccttga aaacttcctc 1320
aagtcaaaag tcagcacctg cttgctagtc acggacccca gaagccagca cacagtacgc 1380
ctcccgcaca agctgccggg catgcactac agtgccaacg agcagtgcca gatcctgttt 1440
ggcatgaatg ccaccttctg cagaaacatg gagcatctaa tgtgtgctgg actgtggtgc 1500
ctggtagaag gagacacatc ctgcaagacc aagctggacc ctcccctgga tggcaccgag 1560
tgtggggcag acaagtggtg ccgcgcgggg gagtgcgtga gcaagacgcc catcccggag 1620
catgtggacg gagactggag cccgtggggc gcctggagca tgtgcagccg aacatgtggg 1680
acgggagccc gcttccggca gaggaaatgt gacaaccccc cccctgggcc tggaggcaca 1740
cactgcccgg gtgccagtgt agaacatgcg gtntgcgaga acctgccctg ccccaagggt 1800
ctgcccagct tccgggacca gcagtgccag gcacacgacc ggctgagccc caagaagaaa 1860
ggcctgctga cagccgtggt ggttgacgat aagccatgtg aactctactg ctcgcccctc 1920
gggaaggagt ccccactgct ggtggccgac agggtcctgg acggtacacc ctgcgggccc 1980
tacgagactg atctctgcgt gcacggcaag tgccagaaaa tcggctgtga cggcatcatc 2040
gggtctgcag ccaaagagga cagatgcggg gtctgcagcg gggacggcaa gacctgccac 2100
ttggtgaagg gcgacttcag ccacgcccgg gggacaggtt atatcgaagc tgccgtcatt 2160
cctgctggag ctcggaggat ccgtgtggtg gaggataaac ctgcccacag ctttctgggt 2220
aaaacacaaa tgact 2235

<210> 2
<211> 1905
<212> DNA
<213> Human

<400> 2
atgtgtgacg gcgccctgct gcctccgctc gtcctgcccg tgctgctgct gctggtttgg 60
ggactggacc cgggcacagc tgtcggcgac gcggcggccg acgtggaggt ggtgctcccg 120
tggcgggtgc gccccgacga cgtgcacctg ccgccgctgc ccgcagcccc cgggccccga 180
cggcggcgac gcccccgcac gcccccagcc gccccgcgcg cccggcccgg agagcgcgcc 240
ctgctgctgc acctgccggc cttcgggcgc gacctgtacc ttcagctgcg ccgcgacctg 300
cgcttcctgt cccgaggctt cgaggtggag gaggcgggcg cggcccggcg ccgcggccgc 360
cccgccgagc tgtgcttcta ctcgggccgt gtgctcggcc accccggctc cctcgtctcg 420
ctcagcgcct gcggcgccgc cggcggcctg gttggcctca ttcagcttgg gcaggagcag 480
gtgctaatcc agcccctcaa caactcccag ggcccattca gtggacgaga acatctgatc 540
aggcgcaaat ggtccttgac ccccagccct tctgctgagg cccagagacc tgagcagctc 600
tgcaaggttc taacagaaaa gaagaagccg acgtggggca ggccttcgcg ggactggcgg 660
gagcggagga acgctatccg gctcaccagc gagcacacgg tggagaccct ggtggtggcc 720
gacgccgaca tggtgcagta ccacggggcc gaggccgccc agaggttcat cctgaccgtc 780
atgaacatgg tatacaatat gtttcagcac cagagcctgg ggattaaaat taacattcaa 840
gtgaccaagc ttgtcctgct acgacaacgt cccgctaagt tgtccattgg gcaccatggt 900
gagcggtccc tggagagctt ctgtcactgg cagaacgagg agtatggagg ggcgcgatac 960
ctcggcaata accaggttcc cggcgggaag gacgacccgc ccctggtgga tgctgccgtg 1020
tttgtgacca ggacagattt ctgtgtacac aaggatgaac cgtgtgacac tgttggaatt 1080
gcttacttag gaggtgtgtg cagtgctaag aggaagtgtg tgcttgccga agacaatggt 1140
ctcaatttgg cctttaccat cgcccatgag ctgggccaca acttgggcat gaaccacgac 1200
gatgaccact catcttgcgc tggcaggtcc cacatcatgt caggagagtg ggtgaaaggc 1260
cggaacccaa gtgacctctc ttggtcctcc tgcagccgag atgaccttga aaacttcctc 1320
aaccctgggc ctggaggcac acactgcccg ggtgccagtg tagaacatgc ggtctgcgag 1380
aacctgccct gccccaaggg tctgcccagc ttccgggacc agcagtgcca ggcacacgac 1440
cggctgagcc ccaagaagaa aggcctgctg acagccgtgg tggttgacga taagccatgt 1500
gaactctact gctcgcccct cgggaaggag tccccactgc tggtggccga cagggtcctg 1560
gacggtacac cctgcgggcc ctacgagact gatctctgcg tgcacggcaa gtgccagaaa 1620
atcggctgtg acggcatcat cgggtctgca gccaaagagg acagatgcgg ggtctgcagc 1680
ggggacggca agacctgcca cttggtgaag ggcgacttca gccacgcccg ggggacagtt 1740
aagaatgatc tctgtacgaa ggtatccaca tgtgtgatgg cagaggctgt tcccaagtgt 1800
ttctcatgtt atatcgaagc tgccgtcatt cctgctggag ctcggaggat ccgtgtggtg 1860
gaggataaac ctgcccacag ctttctgggt aaaacacaaa tgact 1905

<210> 3
<211> 1698
<212> DNA
<213> Human

<400> 3
atgtgtgacg gcgccctgct gcctccgctc gtcctgcccg tgctgctgct gctggtttgg 60
ggactggacc cgggcacaga aaagaagaag ccgacgtggg gcaggccttc gcgggactgg 120
cgggagcgga ggaacgctat ccggctcacc agcgagcaca cggtggagac cctggtggtg 180
gccgacgccg acatggtgca gtaccacggg gccgaggccg cccagaggtt catcctgacc 240
gtcatgaaca tggtatacaa tatgtttcag caccagagcc tggggattaa aattaacatt 300
caagtgacca agcttgtcct gctacgacaa cgtcccgcta agttgtccat tgggcaccat 360
ggtgagcggt ccctggagag cttctgtcac tggcagaaca aggagtatgg aggagcgcga 420
tacctcggca ataaccaggt tcccggcggg aaggacgacc cgcccctggt ggatgctgct 480
gtgtttgtga ccaggacaga tttctgtgta cacaaagatg aaccgtgtga cactgttgga 540
attgcttact taggaggtgt gtgcagtgct aagaggaagt gtgtgcttgc cgaagacaat 600
ggtctcaatt tggcctttac catcgcccat gagctgggcc acaacttggg catgaaccac 660
gacgatgacc actcatcttg cgctggcagg tcccacatca tgtcaggaga gtgggtgaaa 720

ggccggaacc caagtgacct ctcttggtcc tcctgcagcc gagatgacct tgaaaacttc 780
ctcaagtcaa aagtcagcac ctgcttgcta gtcacggacc ccagaagcca gcacacagta 840
cgcctcccgc acaagctgcc gggcatgcac tacagtgcca acgagcagtg ccagatcctg 900
tttggcatga atgccacctt ctgcagaaac atggagcatc taatgtgtgc tggactgtgg 960
tgcctggtag aaggagacac atcctgcaag accaagctgg accctcccct ggatggcacc 1020
gagtgtgggg cagacaagtg gtgccgcgcg ggggagtgcg tgagcaagac gcccatcccg 1080
gagcatgtgg acggagactg gagcccgtgg ggcgcctgga gcatgtgcag ccgaacatgt 1140
gggacgggag cccgcttccg gcagaggaaa tgtgacaacc ccccccctgg gcctggaggc 1200
acacactgcc cgggtgccag tgtagaacat gcggtctgcg agaacctgcc ctgccccaag 1260
ggtctgccca gcttccggga ccagcagtgc caggcacacg accggctgag ccccaagaag 1320
aaaggcctgc tgacagccgt ggtggttgac gataagccat gtgaactcta ctgctcgccc 1380
ctcgggaagg agtccccact gctggtggcc gacagggtcc tggacggtac accctgcggg 1440
ccctacgaga ctgatctctg cgtgcacggc aagtgccaga aaatcggctg tgacggcatc 1500
atcgggtctg cagccaaaga ggacagatgc ggggtctgca gcggggacgg caagacctgc 1560
cacttggtga agggcgactt cagccacgcc cgggggacag gttatatcga agctgccgtc 1620
attcctgctg gagctcggag gatccgtgtg gtggaggata aacctgccca cagctttctg 1680
ggtaaaacac aaatgact 1698

<210> 4
<211> 2675
<212> DNA
<213> Human

<400> 4
atgtgtgacg gcgccctgct gcctccgctc gtcctgcccg tgctgctgct gctggtttgg 60
ggactggacc cgggcacagc tgtcggcgac gcggcggccg acgtggaggt ggtgctcccg 120
tggcgggtgc gccccgacga cgtgcacctg ccgccgctgc ccgcagcccc cgggccccga 180
cggcggcgac gcccccgcac gcccccagcc gccccgcgcg cccggcccgg agagcgcgcc 240
ctgctgctgc acctgccggc cttcgggcgc gacctgtacc ttcagctgcg ccgcgacctg 300
cgcttcctgt cccgaggctt cgaggtggag gaggcgggcg cggcccggcg ccgcggccgc 360
cccgccgagc tgtgcttcta ctcgggccgt gtgctcggcc accccggctc cctcgtctcg 420
ctcagcgcct gcggcgccgc cggcggcctg gttggcctca ttcagcttgg gcaggagcag 480
gtgctaatcc agcccctcaa caactcccag ggcccattca gtggacgaga acatctgatc 540
aggcgcaaat ggtccttgac ccccagccct tctgctgagg cccagagacc tgagcagctc 600
tgcaaggttc taacagaaaa gaagaagccg acgtggggca ggcctttgcg ggactggcgg 660
gagcggagga acgctatccg gctcaccagc gagcacacgg tggagaccct ggtggtggcc 720
gacgccgaca tggtgcagta ccacggggcc gaggctgccc agaggttcat cctgaccgtc 780
atgaacatgg tatacaatat gtttcagcac cagagcctgg ggattaaaat taacattcaa 840
gtgaccaagc ttgtcctgct acgacaacgt cccgctaagt tgtccattgg gcaccatggt 900
gagcggtccc tggagagctt cgtcactggc agaacgagga gtatggagga gcgcgatacc 960
tcggcaataa ccaggttccc ggcgggaagg acgacccgcc cctggtggat gctgctgtgt 1020
ttgtgaccag gacagatttc tgtgtacaca aagatgaacc gtgtgacact gttggaattg 1080
cttacttagg aggtgtgtgc agtgctaaga ggaagtgtgt gcttgccgaa gacaatggtc 1140
tcaatttggc ctttaccatc gcccatgagc tgggccacaa cttgggcatg aaccacgacg 1200
atgaccactc atcttgcgct ggcaggtccc acatcatgtc aggagagtgg gtgaaaggcc 1260
ggaacccaag tgacctctct tggtcctcct gcagccgaga tgaccttgaa aacttcctca 1320
agtcaaaagt cagcacctgc ttgctagtca cggaccccag aagccagcac acagtacgcc 1380
tcccgcacaa gctgccgggc atgcactaca gtgccaacga gcagtgccag atcctgtttg 1440
gcatgaatgc caccttctgc agaaacatgg agcatctaat gtgtgctgga ctgtggtgcc 1500
tggtagaagg agacacatcc tgcaagacca agctggaccc tcccctggat ggcaccgagt 1560
gtggggcaga caagtggtgc cgcgcggggg agtgcgtgag caagacgccc atcccggagc 1620
atgtggacgg agactggagc ccgtggggcg cctggagcat gtgcagccga acatgtggga 1680
cgggagcccg cttccggcag aggaaatgtg acaacccccc ccctgggcct ggaggcacac 1740
actgcccggg tgccagtgta gaacatgcgg tctgcgagaa cctgccctgc cccaagggtc 1800
tgcccagctt ccgggaccag cagtgccagg cacacgaccg gctgagcccc aagaagaaag 1860
gcctgctgac agccgtggtg gttgacgata agccatgtga actctactgc tcgcccctcg 1920
ggaaggagtc cccactgctg gtggccgaca gggtcctgga cggtacaccc tgcgggccct 1980
acgagactga tctctgcgtg cacggcaagt gccagaaaat cggctgtgac ggcatcatcg 2040
ggtctgcagc caaagaggac agatgcgggg tctgcagcgg ggacggcaag acctgccact 2100

tggtgaaggg cgacttcagc cacgcccggg ggacaggtta tatcgaagct gccgtcattc 2160
ctgctggagc tcggaggatc cgtgtggtgg aggataaacc tgcccacagc tttctggctc 2220
tcaaagactc gggtaagggg tccatcaaca gtgactggaa gatagagctc cccggagagt 2280
tccagattgc aggcacaact gttcgctatg tgagaagggg gctgtgggag aagatctctg 2340
ccaagggacc aaccaaacta ccgctgcact tgatggtgtt gttatttcac gaccaagatt 2400
atggaattca ttatgaatac actgttcctg taaaccgcac tgcggaaaat caaagcgaac 2460
cagaaaaacc gcaggactct ttgttcatct ggacccacag cggctgggaa gggtgcagtg 2520
tgcagtgcgg cggaggggag cgcagaacca tcgtttcgtg tacacggatt gtcaacaaga 2580
ccccaacttt ggtgaacgac agtgactgcc ctcaagcaag ccgcccagag ccccaggtcc 2640
gaaggtgcaa cttgcacccc tgccagtcac ggtaa 2675

<210> 5
<211> 745
<212> PRT
<213> Human

<400> 5
Met Cys Asp Gly Ala Leu Leu Pro Pro Leu Val Leu Pro Val Leu Leu
1               5                   10                  15

Leu Leu Val Trp Gly Leu Asp Pro Gly Thr Ala Val Gly Asp Ala Ala
            20                  25                  30

Ala Asp Val Glu Val Val Leu Pro Trp Arg Val Arg Pro Asp Asp Val
        35                  40                  45

His Leu Pro Pro Leu Pro Ala Ala Pro Gly Pro Arg Arg Arg Arg Arg
    50                  55                  60

Pro Arg Thr Pro Pro Ala Ala Pro Arg Ala Arg Pro Gly Glu Arg Ala
65                  70                  75                  80

Leu Leu Leu His Leu Pro Ala Phe Gly Arg Asp Leu Tyr Leu Gln Leu
                85                  90                  95

Arg Arg Asp Leu Arg Phe Leu Ser Arg Gly Phe Glu Val Glu Glu Ala
            100                 105                 110

Gly Ala Ala Arg Arg Arg Gly Arg Pro Ala Glu Leu Cys Phe Tyr Ser
        115                 120                 125

Gly Arg Val Leu Gly His Pro Gly Ser Leu Val Ser Leu Ser Ala Cys
    130                 135                 140

Gly Ala Ala Gly Gly Leu Val Gly Leu Ile Gln Leu Gly Gln Glu Gln
145                 150                 155                 160

Val Leu Ile Gln Pro Leu Asn Asn Ser Gln Gly Pro Phe Ser Gly Arg
                165                 170                 175

Glu His Leu Ile Arg Arg Lys Trp Ser Leu Thr Pro Ser Pro Ser Ala
            180                 185                 190

Glu Ala Gln Arg Pro Glu Gln Leu Cys Lys Val Leu Thr Glu Lys Lys
        195                 200                 205

Lys Pro Thr Trp Gly Arg Pro Ser Arg Asp Trp Arg Glu Arg Arg Asn
    210                 215                 220

Ala Ile Arg Leu Thr Ser Glu His Thr Val Glu Thr Leu Val Val Ala
225                 230                 235                 240

Asp Ala Asp Met Val Gln Tyr His Gly Ala Glu Ala Ala Gln Arg Phe
                245                 250                 255

Ile Leu Thr Val Met Asn Met Val Tyr Asn Met Phe Gln His Gln Ser
            260                 265                 270

Leu Gly Ile Lys Ile Asn Ile Gln Val Thr Lys Leu Val Leu Leu Arg
        275                 280                 285

Gln Arg Pro Ala Lys Leu Ser Ile Gly His His Gly Glu Arg Ser Leu
    290                 295                 300

Glu Ser Phe Cys His Trp Gln Asn Glu Glu Tyr Gly Gly Ala Arg Tyr
305                 310                 315                 320

Leu Gly Asn Asn Gln Val Pro Gly Gly Lys Asp Asp Pro Pro Leu Val
                325                 330                 335

Asp Ala Ala Val Phe Val Thr Arg Thr Asp Phe Cys Val His Lys Asp
            340                 345                 350

Glu Pro Cys Asp Thr Val Gly Ile Ala Tyr Leu Gly Gly Val Cys Ser
        355                 360                 365

Ala Lys Arg Lys Cys Val Leu Ala Glu Asp Asn Gly Leu Asn Leu Ala
    370                 375                 380

Phe Thr Ile Ala His Glu Leu Gly His Asn Leu Gly Met Asn His Asp
385                 390                 395                 400

Asp Asp His Ser Ser Cys Ala Gly Arg Ser His Ile Met Ser Gly Glu
                405                 410                 415

Trp Val Lys Gly Arg Asn Pro Ser Asp Leu Ser Trp Ser Ser Cys Ser
            420                 425                 430

Arg Asp Asp Leu Glu Asn Phe Leu Lys Ser Lys Val Ser Thr Cys Leu
        435                 440                 445

Leu Val Thr Asp Pro Arg Ser Gln His Thr Val Arg Leu Pro His Lys
    450                 455                 460

Leu Pro Gly Met His Tyr Ser Ala Asn Glu Gln Cys Gln Ile Leu Phe
465                 470                 475                 480

Gly Met Asn Ala Thr Phe Cys Arg Asn Met Glu His Leu Met Cys Ala
                485                 490                 495

Gly Leu Trp Cys Leu Val Glu Gly Asp Thr Ser Cys Lys Thr Lys Leu
            500                 505                 510

Asp Pro Pro Leu Asp Gly Thr Glu Cys Gly Ala Asp Lys Trp Cys Arg
        515                 520                 525

Ala Gly Glu Cys Val Ser Lys Thr Pro Ile Pro Glu His Val Asp Gly
    530                 535                 540

Asp Trp Ser Pro Trp Gly Ala Trp Ser Met Cys Ser Arg Thr Cys Gly
545                 550                 555                 560

Thr Gly Ala Arg Phe Arg Gln Arg Lys Cys Asp Asn Pro Pro Pro Gly
                565                 570                 575

Pro Gly Gly Thr His Cys Pro Gly Ala Ser Val Glu His Ala Val Cys
            580                 585                 590

Glu Asn Leu Pro Cys Pro Lys Gly Leu Pro Ser Phe Arg Asp Gln Gln
        595                 600                 605

Cys Gln Ala His Asp Arg Leu Ser Pro Lys Lys Lys Gly Leu Leu Thr
    610                 615                 620

Ala Val Val Val Asp Asp Lys Pro Cys Glu Leu Tyr Cys Ser Pro Leu
625                 630                 635                 640

Gly Lys Glu Ser Pro Leu Leu Val Ala Asp Arg Val Leu Asp Gly Thr
                645                 650                 655

Pro Cys Gly Pro Tyr Glu Thr Asp Leu Cys Val His Gly Lys Cys Gln
            660                 665                 670

Lys Ile Gly Cys Asp Gly Ile Ile Gly Ser Ala Ala Lys Glu Asp Arg
        675                 680                 685

Cys Gly Val Cys Ser Gly Asp Gly Lys Thr Cys His Leu Val Lys Gly
    690                 695                 700

Asp Phe Ser His Ala Arg Gly Thr Gly Tyr Ile Glu Ala Ala Val Ile
705                 710                 715                 720

Pro Ala Gly Ala Arg Arg Ile Arg Val Val Glu Asp Lys Pro Ala His
                725                 730                 735

Ser Phe Leu Gly Lys Thr Gln Met Thr
            740                 745


<210> 6
<211> 635
<212> PRT
<213> Human

<400> 6
Met Cys Asp Gly Ala Leu Leu Pro Pro Leu Val Leu Pro Val Leu Leu
  1               5                  10                  15

Leu Leu Val Trp Gly Leu Asp Pro Gly Thr Ala Val Gly Asp Ala Ala
             20                  25                  30

Ala Asp Val Glu Val Val Leu Pro Trp Arg Val Arg Pro Asp Asp Val
         35                  40                  45

His Leu Pro Pro Leu Pro Ala Ala Pro Gly Pro Arg Arg Arg Arg Arg
     50                  55                  60

Pro Arg Thr Pro Pro Ala Ala Pro Arg Ala Arg Pro Gly Glu Arg Ala
 65                  70                  75                  80

Leu Leu Leu His Leu Pro Ala Phe Gly Arg Asp Leu Tyr Leu Gln Leu
                85                  90                  95

Arg Arg Asp Leu Arg Phe Leu Ser Arg Gly Phe Glu Val Glu Glu Ala
            100                 105                 110

Gly Ala Ala Arg Arg Arg Gly Arg Pro Ala Glu Leu Cys Phe Tyr Ser
        115                 120                 125

Gly Arg Val Leu Gly His Pro Gly Ser Leu Val Ser Leu Ser Ala Cys
    130                 135                 140

Gly Ala Ala Gly Gly Leu Val Gly Leu Ile Gln Leu Gly Gln Glu Gln
145                 150                 155                 160

Val Leu Ile Gln Pro Leu Asn Asn Ser Gln Gly Pro Phe Ser Gly Arg
                165                 170                 175

Glu His Leu Ile Arg Arg Lys Trp Ser Leu Thr Pro Ser Pro Ser Ala
            180                 185                 190

Glu Ala Gln Arg Pro Glu Gln Leu Cys Lys Val Leu Thr Glu Lys Lys
        195                 200                 205

Lys Pro Thr Trp Gly Arg Pro Ser Arg Asp Trp Arg Glu Arg Arg Asn
    210                 215                 220

Ala Ile Arg Leu Thr Ser Glu His Thr Val Glu Thr Leu Val Val Ala
225                 230                 235                 240

Asp Ala Asp Met Val Gln Tyr His Gly Ala Glu Ala Ala Gln Arg Phe
                245                 250                 255

Ile Leu Thr Val Met Asn Met Val Tyr Asn Met Phe Gln His Gln Ser
            260                 265                 270

Leu Gly Ile Lys Ile Asn Ile Gln Val Thr Lys Leu Val Leu Leu Arg
        275                 280                 285

Gln Arg Pro Ala Lys Leu Ser Ile Gly His His Gly Glu Arg Ser Leu
    290                 295                 300

Glu Ser Phe Cys His Trp Gln Asn Glu Glu Tyr Gly Gly Ala Arg Tyr
305                 310                 315                 320

Leu Gly Asn Asn Gln Val Pro Gly Gly Lys Asp Asp Pro Pro Leu Val
                325                 330                 335

Asp Ala Ala Val Phe Val Thr Arg Thr Asp Phe Cys Val His Lys Asp
            340                 345                 350

Glu Pro Cys Asp Thr Val Gly Ile Ala Tyr Leu Gly Gly Val Cys Ser
        355                 360                 365

Ala Lys Arg Lys Cys Val Leu Ala Glu Asp Asn Gly Leu Asn Leu Ala
    370                 375                 380

Phe Thr Ile Ala His Glu Leu Gly His Asn Leu Gly Met Asn His Asp
385                 390                 395                 400

Asp Asp His Ser Ser Cys Ala Gly Arg Ser His Ile Met Ser Gly Glu
                405                 410                 415

Trp Val Lys Gly Arg Asn Pro Ser Asp Leu Ser Trp Ser Ser Cys Ser
            420                 425                 430

Arg Asp Asp Leu Glu Asn Phe Leu Asn Pro Gly Pro Gly Gly Thr His
        435                 440                 445

Cys Pro Gly Ala Ser Val Glu His Ala Val Cys Glu Asn Leu Pro Cys
    450                 455                 460

Pro Lys Gly Leu Pro Ser Phe Arg Asp Gln Gln Cys Gln Ala His Asp
465                 470                 475                 480

Arg Leu Ser Pro Lys Lys Lys Gly Leu Leu Thr Ala Val Val Val Asp
                485                 490                 495

Asp Lys Pro Cys Glu Leu Tyr Cys Ser Pro Leu Gly Lys Glu Ser Pro
            500                 505                 510

Leu Leu Val Ala Asp Arg Val Leu Asp Gly Thr Pro Cys Gly Pro Tyr
        515                 520                 525

Glu Thr Asp Leu Cys Val His Gly Lys Cys Gln Lys Ile Gly Cys Asp
    530                 535                 540

Gly Ile Ile Gly Ser Ala Ala Lys Glu Asp Arg Cys Gly Val Cys Ser
545                 550                 555                 560

Gly Asp Gly Lys Thr Cys His Leu Val Lys Gly Asp Phe Ser His Ala
                565                 570                 575

Arg Gly Thr Val Lys Asn Asp Leu Cys Thr Lys Val Ser Thr Cys Val
            580                 585                 590

Met Ala Glu Ala Val Pro Lys Cys Phe Ser Cys Tyr Ile Glu Ala Ala
        595                 600                 605

Val Ile Pro Ala Gly Ala Arg Arg Ile Arg Val Val Glu Asp Lys Pro
    610                 615                 620

Ala His Ser Phe Leu Gly Lys Thr Gln Met Thr
625                 630                 635


<210> 7
<211> 566
<212> PRT
<213> Human

<400> 7
Met Cys Asp Gly Ala Leu Leu Pro Pro Leu Val Leu Pro Val Leu Leu
  1               5                  10                  15

Leu Leu Val Trp Gly Leu Asp Pro Gly Thr Glu Lys Lys Lys Pro Thr
             20                  25                  30

Trp Gly Arg Pro Ser Arg Asp Trp Arg Glu Arg Arg Asn Ala Ile Arg

35                  40                  45

Leu Thr Ser Glu His Thr Val Glu Thr Leu Val Val Ala Asp Ala Asp
    50                  55                  60

Met Val Gln Tyr His Gly Ala Glu Ala Ala Gln Arg Phe Ile Leu Thr
65                  70                  75                  80

Val Met Asn Met Val Tyr Asn Met Phe Gln His Gln Ser Leu Gly Ile
                85                  90                  95

Lys Ile Asn Ile Gln Val Thr Lys Leu Val Leu Leu Arg Gln Arg Pro
            100                 105                 110

Ala Lys Leu Ser Ile Gly His His Gly Glu Arg Ser Leu Glu Ser Phe
        115                 120                 125

Cys His Trp Gln Asn Lys Glu Tyr Gly Gly Ala Arg Tyr Leu Gly Asn
    130                 135                 140

Asn Gln Val Pro Gly Gly Lys Asp Asp Pro Pro Leu Val Asp Ala Ala
145                 150                 155                 160

Val Phe Val Thr Arg Thr Asp Phe Cys Val His Lys Asp Glu Pro Cys
                165                 170                 175

Asp Thr Val Gly Ile Ala Tyr Leu Gly Gly Val Cys Ser Ala Lys Arg
            180                 185                 190

Lys Cys Val Leu Ala Glu Asp Asn Gly Leu Asn Leu Ala Phe Thr Ile
        195                 200                 205

Ala His Glu Leu Gly His Asn Leu Gly Met Asn His Asp Asp Asp His
    210                 215                 220

Ser Ser Cys Ala Gly Arg Ser His Ile Met Ser Gly Glu Trp Val Lys
225                 230                 235                 240

Gly Arg Asn Pro Ser Asp Leu Ser Trp Ser Ser Cys Ser Arg Asp Asp
                245                 250                 255

Leu Glu Asn Phe Leu Lys Ser Lys Val Ser Thr Cys Leu Leu Val Thr
            260                 265                 270

Asp Pro Arg Ser Gln His Thr Val Arg Leu Pro His Lys Leu Pro Gly
        275                 280                 285

Met His Tyr Ser Ala Asn Glu Gln Cys Gln Ile Leu Phe Gly Met Asn
    290                 295                 300

Ala Thr Phe Cys Arg Asn Met Glu His Leu Met Cys Ala Gly Leu Trp
305                 310                 315                 320

Cys Leu Val Glu Gly Asp Thr Ser Cys Lys Thr Lys Leu Asp Pro Pro
                325                 330                 335

Leu Asp Gly Thr Glu Cys Gly Ala Asp Lys Trp Cys Arg Ala Gly Glu
            340                 345                 350

Cys Val Ser Lys Thr Pro Ile Pro Glu His Val Asp Gly Asp Trp Ser

355                 360                 365

Pro Trp Gly Ala Trp Ser Met Cys Ser Arg Thr Cys Gly Thr Gly Ala
    370                 375                 380

Arg Phe Arg Gln Arg Lys Cys Asp Asn Pro Pro Pro Gly Pro Gly Gly
385                 390                 395                 400

Thr His Cys Pro Gly Ala Ser Val Glu His Ala Val Cys Glu Asn Leu
                405                 410                 415

Pro Cys Pro Lys Gly Leu Pro Ser Phe Arg Asp Gln Gln Cys Gln Ala
            420                 425                 430

His Asp Arg Leu Ser Pro Lys Lys Lys Gly Leu Leu Thr Ala Val Val
        435                 440                 445

Val Asp Asp Lys Pro Cys Glu Leu Tyr Cys Ser Pro Leu Gly Lys Glu
    450                 455                 460

Ser Pro Leu Leu Val Ala Asp Arg Val Leu Asp Gly Thr Pro Cys Gly
465                 470                 475                 480

Pro Tyr Glu Thr Asp Leu Cys Val His Gly Lys Cys Gln Lys Ile Gly
                485                 490                 495

Cys Asp Gly Ile Ile Gly Ser Ala Ala Lys Glu Asp Arg Cys Gly Val
            500                 505                 510

Cys Ser Gly Asp Gly Lys Thr Cys His Leu Val Lys Gly Asp Phe Ser
        515                 520                 525

His Ala Arg Gly Thr Gly Tyr Ile Glu Ala Ala Val Ile Pro Ala Gly
    530                 535                 540

Ala Arg Arg Ile Arg Val Val Glu Asp Lys Pro Ala His Ser Phe Leu
545                 550                 555                 560

Gly Lys Thr Gln Met Thr
                565


<210> 8
<211> 891
<212> PRT
<213> Human

<400> 8
Met Cys Asp Gly Ala Leu Leu Pro Pro Leu Val Leu Pro Val Leu Leu
1               5                   10                  15

Leu Leu Val Trp Gly Leu Asp Pro Gly Thr Ala Val Gly Asp Ala Ala
            20                  25                  30

Ala Asp Val Glu Val Val Leu Pro Trp Arg Val Arg Pro Asp Asp Val
        35                  40                  45

His Leu Pro Pro Leu Pro Ala Ala Pro Gly Pro Arg Arg Arg Arg Arg
    50                  55                  60

Pro Arg Thr Pro Pro Ala Ala Pro Arg Ala Arg Pro Gly Glu Arg Ala
65                  70                  75                  80

Leu Leu Leu His Leu Pro Ala Phe Gly Arg Asp Leu Tyr Leu Gln Leu
                85                  90                  95

Arg Arg Asp Leu Arg Phe Leu Ser Arg Gly Phe Glu Val Glu Glu Ala
            100                 105                 110

Gly Ala Ala Arg Arg Arg Gly Arg Pro Ala Glu Leu Cys Phe Tyr Ser
        115                 120                 125

Gly Arg Val Leu Gly His Pro Gly Ser Leu Val Ser Leu Ser Ala Cys
    130                 135                 140

Gly Ala Ala Gly Gly Leu Val Gly Leu Ile Gln Leu Gly Gln Glu Gln
145                 150                 155                 160

Val Leu Ile Gln Pro Leu Asn Asn Ser Gln Gly Pro Phe Ser Gly Arg
                165                 170                 175

Glu His Leu Ile Arg Arg Lys Trp Ser Leu Thr Pro Ser Pro Ser Ala
            180                 185                 190

Glu Ala Gln Arg Pro Glu Gln Leu Cys Lys Val Leu Thr Glu Lys Lys
        195                 200                 205

Lys Pro Thr Trp Gly Arg Pro Leu Arg Asp Trp Arg Glu Arg Arg Asn
    210                 215                 220

Ala Ile Arg Leu Thr Ser Glu His Thr Val Glu Thr Leu Val Val Ala
225                 230                 235                 240

Asp Ala Asp Met Val Gln Tyr His Gly Ala Glu Ala Ala Gln Arg Phe
                245                 250                 255

Ile Leu Thr Val Met Asn Met Val Tyr Asn Met Phe Gln His Gln Ser
            260                 265                 270

Leu Gly Ile Lys Ile Asn Ile Gln Val Thr Lys Leu Val Leu Leu Arg
        275                 280                 285

Gln Arg Pro Ala Lys Leu Ser Ile Gly His His Gly Glu Arg Ser Leu
    290                 295                 300

Glu Ser Phe Cys His Trp Gln Asn Glu Glu Tyr Gly Gly Ala Arg Tyr
305                 310                 315                 320

Leu Gly Asn Asn Gln Val Pro Gly Gly Lys Asp Asp Pro Pro Leu Val
                325                 330                 335

Asp Ala Ala Val Phe Val Thr Arg Thr Asp Phe Cys Val His Lys Asp
            340                 345                 350

Glu Pro Cys Asp Thr Val Gly Ile Ala Tyr Leu Gly Gly Val Cys Ser
        355                 360                 365

Ala Lys Arg Lys Cys Val Leu Ala Glu Asp Asn Gly Leu Asn Leu Ala
    370                 375                 380

Phe Thr Ile Ala His Glu Leu Gly His Asn Leu Gly Met Asn His Asp
385                 390                 395                 400

Asp Asp His Ser Ser Cys Ala Gly Arg Ser His Ile Met Ser Gly Glu
                405                 410                 415

Trp Val Lys Gly Arg Asn Pro Ser Asp Leu Ser Trp Ser Ser Cys Ser
            420                 425                 430

Arg Asp Asp Leu Glu Asn Phe Leu Lys Ser Lys Val Ser Thr Cys Leu
        435                 440                 445

Leu Val Thr Asp Pro Arg Ser Gln His Thr Val Arg Leu Pro His Lys
    450                 455                 460

Leu Pro Gly Met His Tyr Ser Ala Asn Glu Gln Cys Gln Ile Leu Phe
465                 470                 475                 480

Gly Met Asn Ala Thr Phe Cys Arg Asn Met Glu His Leu Met Cys Ala
                485                 490                 495

Gly Leu Trp Cys Leu Val Glu Gly Asp Thr Ser Cys Lys Thr Lys Leu
            500                 505                 510

Asp Pro Pro Leu Asp Gly Thr Glu Cys Gly Ala Asp Lys Trp Cys Arg
        515                 520                 525

Ala Gly Glu Cys Val Ser Lys Thr Pro Ile Pro Glu His Val Asp Gly
    530                 535                 540

Asp Trp Ser Pro Trp Gly Ala Trp Ser Met Cys Ser Arg Thr Cys Gly
545                 550                 555                 560

Thr Gly Ala Arg Phe Arg Gln Arg Lys Cys Asp Asn Pro Pro Pro Gly
                565                 570                 575

Pro Gly Gly Thr His Cys Pro Gly Ala Ser Val Glu His Ala Val Cys
            580                 585                 590

Glu Asn Leu Pro Cys Pro Lys Gly Leu Pro Ser Phe Arg Asp Gln Gln
        595                 600                 605

Cys Gln Ala His Asp Arg Leu Ser Pro Lys Lys Lys Gly Leu Leu Thr
    610                 615                 620

Ala Val Val Val Asp Asp Lys Pro Cys Glu Leu Tyr Cys Ser Pro Leu
625                 630                 635                 640

Gly Lys Glu Ser Pro Leu Leu Val Ala Asp Arg Val Leu Asp Gly Thr
                645                 650                 655

Pro Cys Gly Pro Tyr Glu Thr Asp Leu Cys Val His Gly Lys Cys Gln
            660                 665                 670

Lys Ile Gly Cys Asp Gly Ile Ile Gly Ser Ala Ala Lys Glu Asp Arg
        675                 680                 685

Cys Gly Val Cys Ser Gly Asp Gly Lys Thr Cys His Leu Val Lys Gly
    690                 695                 700

Asp Phe Ser His Ala Arg Gly Thr Gly Tyr Ile Glu Ala Ala Val Ile
705                 710                 715                 720

Pro Ala Gly Ala Arg Arg Ile Arg Val Val Glu Asp Lys Pro Ala His
                725                 730                 735

Ser Phe Leu Ala Leu Lys Asp Ser Gly Lys Gly Ser Ile Asn Ser Asp
            740                 745                 750

Trp Lys Ile Glu Leu Pro Gly Glu Phe Gln Ile Ala Gly Thr Thr Val
        755                 760                 765

Arg Tyr Val Arg Arg Gly Leu Trp Glu Lys Ile Ser Ala Lys Gly Pro
    770                 775                 780

Thr Lys Leu Pro Leu His Leu Met Val Leu Leu Phe His Asp Gln Asp
785                 790                 795                 800

Tyr Gly Ile His Tyr Glu Tyr Thr Val Pro Val Asn Arg Thr Ala Glu
                805                 810                 815

Asn Gln Ser Glu Pro Glu Lys Pro Gln Asp Ser Leu Phe Ile Trp Thr
            820                 825                 830

His Ser Gly Trp Glu Gly Cys Ser Val Gln Cys Gly Gly Gly Glu Arg
        835                 840                 845

Arg Thr Ile Val Ser Cys Thr Arg Ile Val Asn Lys Thr Pro Thr Leu
    850                 855                 860

Val Asn Asp Ser Asp Cys Pro Gln Ala Ser Arg Pro Glu Pro Gln Val
865                 870                 875                 880

Arg Arg Cys Asn Leu His Pro Cys Gln Ser Arg
                885                 890

<210> 9
<211> 1966
<212> DNA
<213> Human

<400> 9
ccccgccgag ctgtgcttct actcgggccg tgtgctcggc caccccggct ccctcgtctc 60
gctcagcgcc tgcggcgccg ccggcggcct ggttggcctc attcagcttg ggcaggagca 120
ggtgctaatc cagcccctca acaactccca gggcccattc agtggacgag aacatctgat 180
caggcgcaaa tggtccttga cccccagccc ttctgctgag gcccagagac ctgagcagct 240
ctgcaaggtt ctaacagaaa agaagaagcc gacgtggggc aggcctttgc gggactggcg 300
ggagcggagg aacgctatcc ggctcaccag cgagcacacg gtggagaccc tggtggtggc 360
cgacgccgac atggtgcagt accacggggc cgaggctgcc cagaggttca tcctgaccgt 420
catgaacatg gtatacaata tgtttcagca ccagagcctg gggattaaaa ttaacattca 480
agtgaccaag cttgtcctgc tacgacaacg tcccgctaag ttgtccattg ggcaccatgg 540
tgagcggtcc ctggagagct tctgtcactg gcagaacgag gagtatggag gagcgcgata 600
cctcggcaat aaccaggttc ccggcgggaa ggacgacccg cccctggtgg atgctgctgt 660
gtttgtgacc aggacagatt tctgtgtaca caaagatgaa ccgtgtgaca ctgttggaat 720
tgcttactta ggaggtgtgt gcagtgctaa gaggaagtgt gtgcttgccg aagacaatgg 780
tctcaatttg gcctttacca tcgcccatga gctgggccac aacttgggca tgaaccacga 840
cgatgaccac tcatcttgcg ctggcaggtc ccacatcatg tcaggagagt gggtgaaagg 900
ccggaaccca agtgacctct cttggtcctc ctgcagccga gatgaccttg aaaacttcct 960
caagtcaaaa gtcagcacct gcttgctagt cacggacccc agaagccagc acacagtacg 1020

cctcccgcac aagctgccgg gcatgcacta cagtgccaac gagcagtgcc agatcctgtt 1080
tggcatgaat gccaccttct gcagaaacat ggagcatcta atgtgtgctg gactgtggtg 1140
cctggtagaa ggagacacat cctgcaagac caagctggac cctcccctgg atggcaccga 1200
gtgtggggca gacaagtggt gccgcgcggg ggagtgcgtg agcaagacgc ccatcccgga 1260
gcatgtggac ggagactgga gcccgtgggg cgcctggagc atgtgcagcc gaacatgtgg 1320
gacgggagcc cgcttccggc agaggaaatg tgacaacccc ccccctgggc ctggaggcac 1380
acactgcccg ggtgccagtg tagaacatgc ggtctgcgag aacctgccct gccccaaggg 1440
tctgcccagc ttccgggacc agcagtgcca ggcacacgac cggctgagcc ccaagaagaa 1500
aggcctgctg acagccgtgg tggttgacga taagccatgt gaactctact gctcgcccct 1560
cgggaaggag tccccactgc tggtggccga cagggtcctg gacggtacac cctgcgggcc 1620
ctacgagact gatctctgcg tgcacggcaa gtgccagaaa atcggctgtg acggcatcat 1680
cgggtctgca gccaaagagg acagatgcgg ggtctgcagc ggggacggca agacctgcca 1740
cttggtgaag ggcgacttca gccacgcccg ggggacaggt tatatcgaag ctgccgtcat 1800
tcctgctgga gctcggagga tccgtgtggt ggaggataaa cctgcccaca gctttctggg 1860
taaaacacaa atgacttgac tcaccattta tgtgttgaga atcgattttg atgatcagtc 1920
tggtaaattg gttcagtgtc aaaaaaaaaa aaaaaaaaaa aaaaaa 1966

<210> 10
<211> 46
<212> DNA
<213> Human

<400> 10
tcacttgtca tcgtcgtcct tgtagtcacc cagaaagctg tgggca 46

<210> 11
<211> 20
<212> DNA
<213> Human

<400> 11
ggacgttgtc gtagcaggac 20

<210> 12
<211> 26
<212> DNA
<213> Human

<400> 12
ggaattcggg taccatgtgt gacggc 26